# EUROPEAN PATENT SPECIFICATION

(11) **EP 2 471 902 B1**
(45) Date of publication and mention of the grant of the patent: **30.05.2018**
(21) Application number: 10811319.2
(22) Date of filing: 25.08.2010
(51) Int. Cl.: A61L 27/26, A61L 27/36, A61L 27/38, A61L 27/60, A61K 35/33, A61K 35/36, A61K 35/51, C12N 5/00, C12N 5/073, C12N 5/079, C12N 5/071

(54) **PRODUCTION OF ARTIFICIAL TISSUES BY MEANS OF TISSUE ENGINEERING USING AGAROSE-FIBRIN BIOMATERIALS**
HERSTELLUNG KÜNSTLICHER GEWEBE DURCH GEWEBE-ENGINEERING MIT AGAROSE-FIBRIN-BIOMATERIALIEN
ÉLABORATION DE TISSUS ARTIFICIELS PAR INGÉNIERIE TISSULAIRE AU MOYEN DE BIOMATÉRIAUX DE FIBRINE ET D'AGAROSE

(30) Priority: 25.08.2009 ES 200930625; 02.11.2009 ES 200930943
(43) Date of publication of application: 04.07.2012
(73) Proprietor: Servicio Andaluz de Salud, 41071 Sevilla (ES); Universidad de Granada, 18071 Granada (ES)
(72) Inventor: ALAMINOS MINGORANCE, Miguel, E-41092 Sevilla (ES); MUÑOZ ÁVILA, José Ignacio, E-41092 Sevilla (ES); GONZÁLEZ ANDRADES, Miguel, E-41092 Sevilla (ES); CAMPOS MUÑOZ, Antonio, E-41092 Sevilla (ES); GARZÓN BELLO, Ingrid Johanna, E-41092 Sevilla (ES)
(74) Representative: ABG Patentes, S.L.
(86) International application number: PCT/ES2010/070569
(87) International publication number: WO 2011/023843

(56) References cited:
- WO-A1-99/56798
- AU-A8- 2006 202 156
- US-A1- 2002 114 796
- US-A1- 2008 131 473
- ALAMINOS, M. ET AL.: 'Construction of a complete rabbit cornea substitute using a fibrin-agarose scaffold.' INVESTIGATIVE OPHTHALMOLOGY & VISUAL SCIENCE. vol. 47, no. 8, August 2006, pages 3311 - 3317, XP008151283
- FRISMAN, I. ET AL.: 'Nanostructuring of PEG-fibrinogen polymeric scaffolds.' ACTA BIOMATERIALIA. vol. 6, no. 7, 15 July 2009, pages 2518 - 2524, XP027052681
- SANCHEZ-QUEVEDO, M. C. ET AL.: 'Histological and histochemical evaluation of human oral mucosa constructs devtheoped by tissue ingineering.' HISTOLOGY AND HISTOPATHOLOGY. vol. 22, no. 6, June 2007, pages 631 - 640, XP008151209
- OSATHANON, T. ET AL.: 'Microporous nanofibrous fibrin-based scaffolds for bone tissue ingineering.' BIOMATERIALS vol. 29, no. 30, October 2008, pages 4091 - 4099, XP024527552
- GONZALEZ-ANDRADES, M. ET AL.: 'Sequential devtheopment of intercthelular junctions in bioengineered human corneas.' JOURNAL OF TISSUE INGINEERING AND REGENERATIVE MEDICINE. vol. 3, no. 6, 05 May 2009, pages 442 - 449, XP008151211
- HASHI, C. K. ET AL.: 'Antithrombogenic property of bone marrow mesenchymal stem cthels in nanofibrous vascular grafts.' PROCEEDINGS OF THE NATIONAL ACADEMY OF SCIENCES OF THE UNITED STATES OF AMERICA. vol. 104, no. 29, 17 July 2007, pages 11915 - 11920, XP008151212
- SHIH YU-RU, V. ET AL.: 'Growth of mesenchymal stem cthels on theectrospun type I collagen nanofibers.' STEM CELLS (DAYTON, OHIO). vol. 24, no. 11, November 2006, pages 2391 - 2397, XP008151226
- PERKA, C. ET AL.: 'Joint cartilage repair with transplantation of embryonic chondrocytes embedded in collagen-fibrin matrices.' CLINICAL AND EXPERIMENTAL RHEUMATOLOGY. vol. 18, no. 1, January 2000, pages 13 - 22, XP008151213

## Description

The present invention is encompassed within the field of biomedicine and more specifically tissue engineering. It relates specifically to an *in vitro* method for preparing an artificial tissue, the artificial tissue obtainable by said method and the use of this artificial tissue to partially or completely increase, restore or replace the functional activity of a damaged tissue or organ.

### Prior State of the Art

Tissue engineering is a group of techniques and disciplines which allows designing and generating artificial tissues in laboratory from stem cells originating from tissue samples obtained from biopsies and therefore involves a great breakthrough in organ transplant and regenerative medicine. Tissue engineering is one of the biotechnology areas that has undergone the greatest development in the recent years due to its potential use for *in vitro* tissue and organ production for implanting in patients needing these tissues. Nevertheless, the artificial tissues described until now have several problems and complications; some of which are stated below, using such as, for example, the artificial tissues from skin, cornea, bladder and urethra.

The urinary bladder is the organ responsible for receiving and storing urine. Located in the pelvic floor, the urinary bladder is characterized by its capacity and distensibility which allows it to store and retain urine. Continence is the result of contracting the urinary sphincter which closes the urethra and the bladder neck preventing urine leakage, as well as the result of relaxing and easing the bladder for storing the urine which will be accumulated therein. Many congenital or acquired pathologies can affect bladder integrity, altering the continence function thereof. On one hand, bladder malformations tend to be associated with serious bladder wall defects requiring urgent surgical repair. On the other hand, pelvic traumas, bladder cancer and traumatic spinal cord injuries are common pathologies requiring the use of extravesical tissues for repairing the damaged bladder. In this context, bladder augmentations are currently carried out using intestine (enterocystoplasty), stomach (gastrocystoplasty) or urothelium (ureterocystoplasty), complications associated with these techniques being very common.

Up until now, very few urinary bladder replacement models having clinical use have been described. Recently (Atala et al. Lancet. 2006 Apr 15;367(9518):1241-6), researchers from the Children's Hospital of Boston successfully implanted an artificial bladder replacement generated from collagen and polyglycolic acid into seven patients with serious bladder damage. However, the artificial bladder models available for treating the patients needing them are very limited and have plenty of drawbacks, including bad quality and the limited manipulability of the tissues generated. Furthermore, collagen is a product that tends to contract and losses volume when it is used in tissue engineering, its consistency being limited and therefore its surgical manipulability.

The urethra is the duct through which the urine stored in the urinary bladder is removed to the outside. In addition to its excreting function in both sexes, the urethra plays a reproductive function in men by allowing the passage of seminal content from the seminal vesicles during ejaculation. There are many congenital conditions (mainly hypospadias and epispadias) or acquired conditions (traumatisms, stenosis, etc.) which affect its functional integrity and which require replacing it to a greater or lesser extent to re-establish its normal function (Baird et al. J Urol. 2005;174:1421-4; Persichetti et al. Plast Reconstr Surg. 2006;117:708-10).

Injured tissues have been traditionally repaired with artificial prosthetic elements or tissues taken from a part of the patient him/herself (autograft or autotransplant) or from another individual (heterologous transplant). Autologous adjacent tissue flaps or free grafts mainly of bladder or oral mucosa are currently resorted to for correcting most of the urethral pathologies. However, it is not always possible to obtain local flaps and the removal from bladder or oral mucosa is not free from complications and side effects both for the donor and the recipient area (Corvin et al. Urologe A. 2004;43(10):1213-6; Schultheiss et al. World J Urol. 2000;18:84-90). On the other hand, the use of heterologous tissues has produced rather poor results in urethra replacement, immunological rejections of the transplanted tissue being very common.

Until now, very few urethra replacement models with probable clinical use have been described, the cases described in the literature wherein a urethral replacement has been implanted in patients being very limited. Most of the models described until now are based on collagen biomaterials (De Filippo et al. J Urol. 2002 Oct;168(4 Pt 2) :1789-92; El-Kassaby et al. J Urol. 2003 Jan;169(1):170-3; discussion 173) or on the skin of the patient him/herself (Lin et al. Zhonghua Yi Xue Za Zhi. 2005 Apr 20;85(15):1057-9). However, all these models have several problems and complications and a urethral replacement free from these problems has yet to be developed. On one hand, the collagen is a product that tends to contract and losses volume when it is used in tissue engineering, its consistency being limited and therefore its surgical manipulability. On the other, the use of autologous skin has not sufficiently demonstrated its capacity to be adapted to the urethra conditions, recelularizing fragments of decelularized dermis being very difficult.

Cornea is a vessel free transparent structure through which the light penetrates into the eye, forming the main barrier of the eyeball with the outer environment. For that reason, the integrity and the correct operation thereof are essential for a correct visual function. Congenital or acquired corneal pathology is one of the most common problems in ophthalmology, there being many causes leading to serious alteration of the physiology and corneal structure. In these cases, aggressive treatments which are not free from complications tend to be resorted to, such as amniotic membrane implants, the different types of keratoprosthesis and even heterologous corneal transplant (keratoplasty). However, corneal transplant is a technique which highly depends on the availability of corneas originating from dead donors, which means that many people remain on the transplant waiting list for lengthy periods of time. On the other hand, it is well known that the organ transplant originating from a donor is subjected to the possibility of immunological rejection when these organs are implanted, forcing the patient to be subjected to an immunosuppressive therapy for his/her whole life. Finally, the transplant of organ or tissue of any type, included the cornea, is a technique subjected to the possibility of transmitting all kind of infectious diseases from the donor to the recipient, including HIV, hepatitis, herpes, bacterial and fungal diseases, etc. All these problems and complications derived from corneal implant make the search for therapeutic alternatives to heterologous transplant necessary.

The production of a corneal replacement (corneal construct or artificial cornea) in laboratory is one of the areas gaining greater importance within tissue engineering, there being many laboratories which are currently attempting without much success to obtain a good quality corneal replacement which can be used in human clinical practice or for evaluating pharmacological and chemical products (Griffith et al. Functional human corneal equivalents constructed from cell lines. Science. 1999;286(5447):2169-72; Orwin et al. Tissue Eng. 2000;6(4):307-19; Reichl et al. Int J Pharm. 2003;250:191-201). In that sense, artificial corneas of animal and human origin have been developed. In both cases, the models developed used different biomaterials such as type I collagen, silk fibroin (Higa and Shimazaki. Cornea. 2008 Sep;27 Suppl 1:S41-7), chitosan (Gao et al. J Mater Sci Mater Med. 2008 Dec;19(12) :3611-9), polyglycolic acid (Hu et al. Tissue Eng. 2005 Nov-Dec;11(11-12):1710-7) and fibrin with agarose (Alaminos et al. Invest Ophthalmol Vis Sci (IOVS). 2006; 47: 3311-3317; Gonzalez-Andrades et al. J Tissue Eng Regen Med. 2009 May 5). Of all these biomaterials, the best results obtained until now are those having fibrin and agarose as the base. The corneas made of type I collagen tend to lose volume and retract with the further drawback that the collagen used is of animal origin. Fibroin and chitosan are products generated from invertebrate animals, which causes significant biocompatibility problems. The corneas developed from fibrin and agarose, in contrast, have the advantage of containing fibrin originating from the blood of the same patient, whereas the agarose forms an inert product from the immunological view point.

The skin is the largest organ of the human body and plays an essential role in maintaining the internal balance, forming the main protective barrier of the organism against any type of external attack. There are many skin pathologies, wounds, pressure ulcers and burns being the most common. Current treatments based on the use of skin flaps or grafts or even on implanting the skin originating from a donor, are associated with several problems.

The need to solve these problems make the search for alternatives based on producing human artificial skin products produced by means of tissue engineering necessary (Horch et al. Burns. 2005 Aug;31(5):597-602). Specifically, up until now different types of artificial skin including synthetic and biological skin covers have been designed, although none of them has successfully reproduced the structure and the functions of the native human skin accurately. On one hand, the synthetic skin covers consist of non-absorbable biomaterials and are free of living cells which can be used as temporary covers or as guided tissue repair inducing agents. These artificial and inert tissues have very little biological activity, therefore they cannot be used in deep or extensive injuries. On the other hand, the biological covers consist of using artificial human skin in which there are living cells and extracellular matrices attempting to reproduce the normal human skin structure. Up until now, the artificial human skin offering the best results is the artificial skin produced by means of tissue engineering from skin stem cells using fibrin originating from the human plasma as the biomaterial (Meana et al. Burns 1998; 24: 621-630; Del Rio et al. Hum Gene Ther. 2002 May 20;13(8) :959-68; Llames et al. Transplantation. 2004 Feb 15;77(3) :350-5; Llames et al. Cell Tissue Bank 2006; 7: 4753.). Although these techniques involved a great breakthrough, their clinical use is limited mainly due to their limited consistency, their difficult manipulation and their extreme fragility. One of the most consistent tissue replacements is that combining the use of fibrin with agarose. Up until now, agarose has been used for generating cartilage replacements (Miyata et al. J Biomech Eng. 2008 Oct;130(5):051016), cornea replacements (Alaminos et al. Invest Ophthalmol Vis Sci (IOVS). 2006; 47: 3311-3317) and human oral mucosa replacement (Alaminos et al. J Tissue Eng Regen Med. 2007 Sep-Oct;1(5) :350-9; Sanchez-Quevedo et al. Histol Histopathol. 2007 Jun;22(6):631-40), but there is no prior experience in the use of this biomaterial for producing artificial skin.

Tissue engineering is one of the areas that is gaining greater importance within biotechnology. However, the drawbacks of the artificial tissues existing until now make developing new techniques which allow obtaining artificial tissues which can be used in human clinical practice or for evaluating pharmacological and chemical products, overcoming the limitations detected until now necessary.

### Summary of the Invention

In a first aspect, the invention relates to an *in vitro* method for preparing an artificial tissue comprising:
a) adding a composition comprising fibrinogen to a sample of isolated cells,
b) adding an antifibrinolytic agent to the product resulting from step (a),
c) adding at least one coagulation factor, a source of calcium, thrombin, or any combination of the above to the product resulting from step (b),
d) adding a composition of a polysaccharide to the product resulting from step (c),
e) culturing isolated cells in or on the product resulting from step (d), and
f) inducing the nanostructuring of the product resulting from step (e), wherein said nanostructuring induction comprises the dehydration and/ or mechanical compression of the product resulting from step (e), and wherein the polysaccharide of step (d) is agarose. In a second aspect, the invention relates to an artificial tissue obtainable by the method of the invention.

In a third aspect, the invention relates to the artificial tissue of the invention for use in medicine.

In a fourth aspect, the invention relates to the artificial tissue of the invention for use in partially or completely increasing, restoring or replacing the functional activity of a diseased or damaged tissue or organ.

In a fifth aspect, the invention relates to a pharmaceutical composition comprising the artificial tissue of the invention.

### Brief Summary of the Drawings

Figure 1 shows a diagram of the method used for the nanostructuring of the artificial tissue.
Figure 2 shows the evaluation of the artificial human skin product. A. *In vitro* microscopy analysis. B. Macro- and microscopic analyses of the artificial human skin evaluated *in vivo.* C. Immunohistochemical analysis.
Figure 3 shows the evaluation of the artificial human skin product with cells of Wharton's jelly. A. Determination of the viability of the Wharton's jelly stem cells. B. Microscopic analysis. C. Immunohistochemical analysis of the following proteins: pancytokeratin (PANC), keratin 1 (KRT1), keratin 10 (KRT10), involucrin (INVOL) and filagrin (F I LAG) .

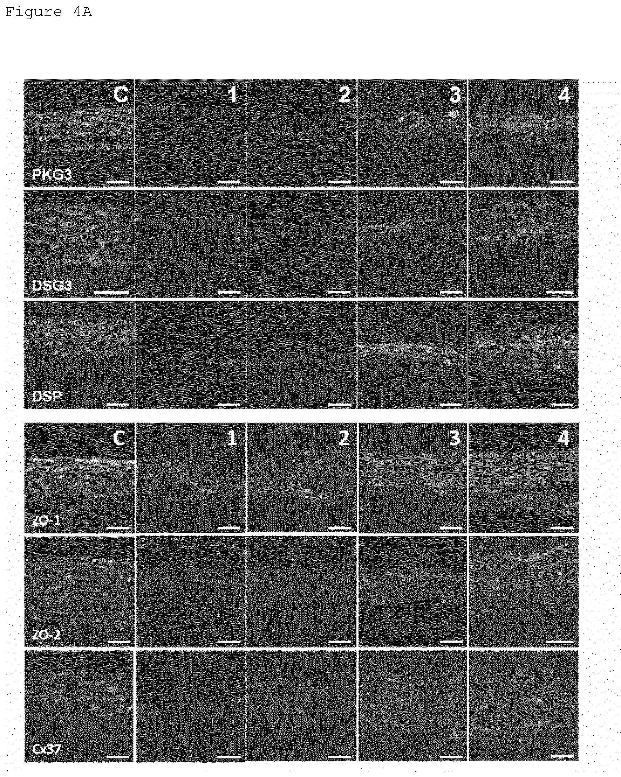

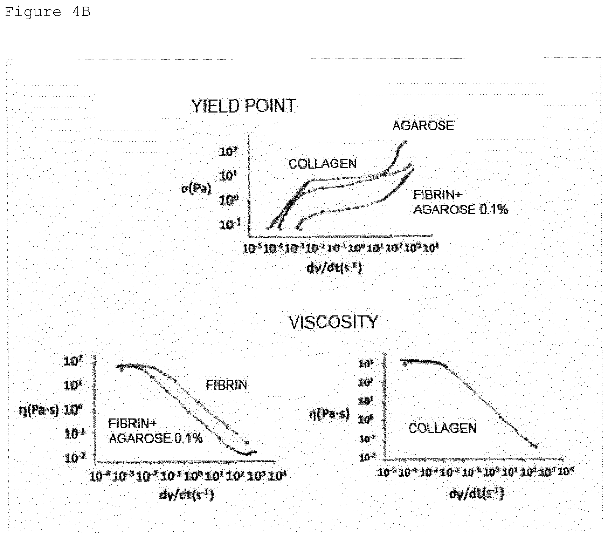

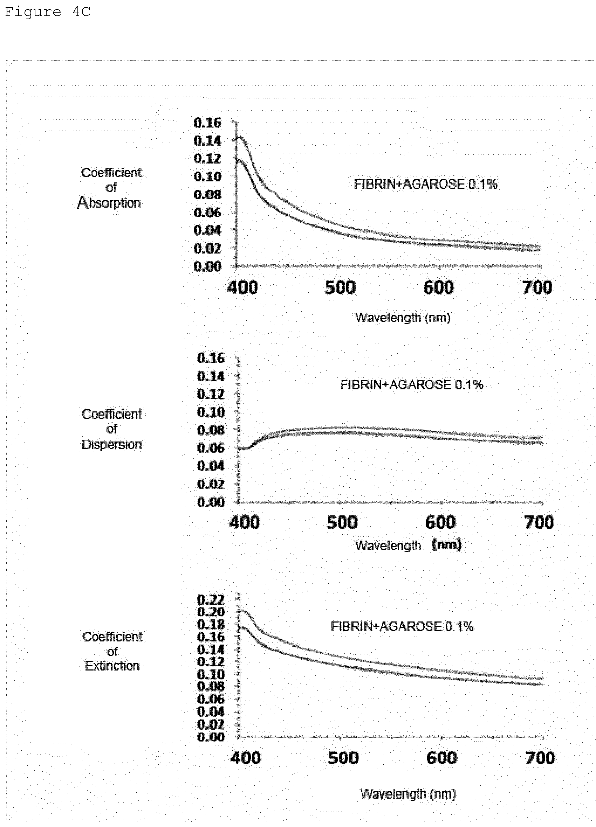

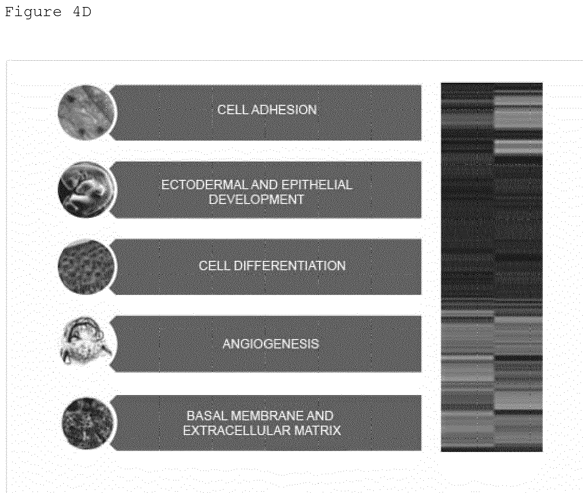

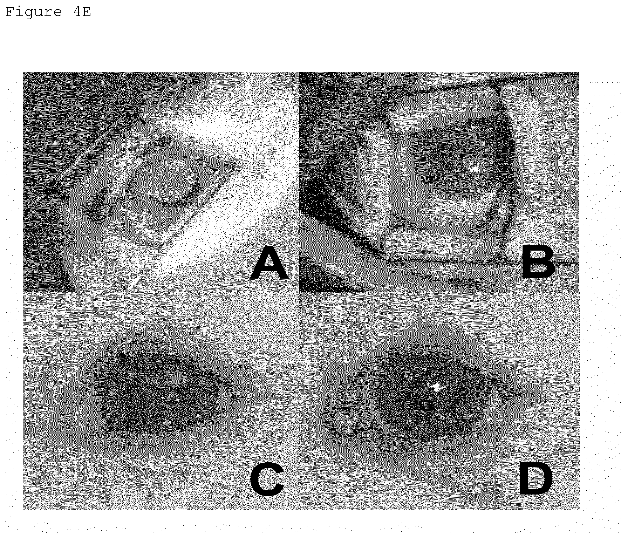

Figure 4 shows the evaluation of the corneal products. A. Microscopic and immunohistochemical analyses: immunofluorescence of different proteins related to intercellular bindings in the epithelium of the human control corneas (C) and of the corneal products kept *in vitro* in different stages of maturation and development (1: cornea with a single epithelium layer, 2: cornea with 2-3 layers, 3: cornea with stratified epithelium, 4: cornea with stratified epithelium and subjected to air-liquid technique). B. Rheological quality control. C. Optical quality control. D. Genetic control: main gene functions expressed by the artificial corneal constructs generated by means of tissue engineering. E. *In vivo* evaluation of the clinical performance of the artificial corneal products in an animal model. A: after removing the front hemicornea, the corneal construct was placed on the surface of the stroma; B: final appearance once sutured; C: evolution after 3 weeks; D: evolution after 6 weeks.
Figure 5 shows the evaluation of the artificial human urethra product. A. Microscopic analysis. B. Immunohistochemical analysis (integrin expression).
Figure 6 shows the evaluation of the artificial urinary bladder product. A. Microscopic analysis: Artificial human bladder produced in laboratory and kept in culture for 1 and 3 weeks, respectively. B. Immunohistochemical analysis: Analysis of the artificial human bladder produced by means of tissue engineering and of the normal control human bladder for cytokeratins (CK) 7, 8, 4, 13 and pancytokeratin by means of immunofluorescence.

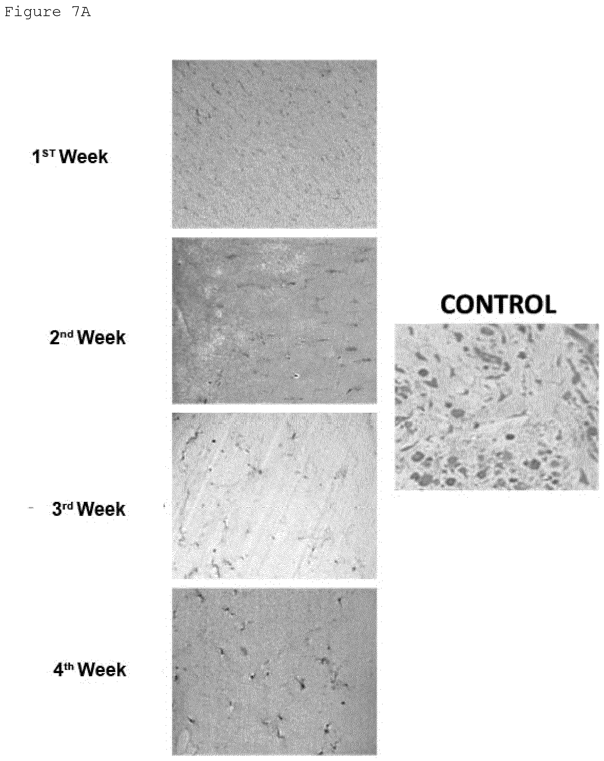

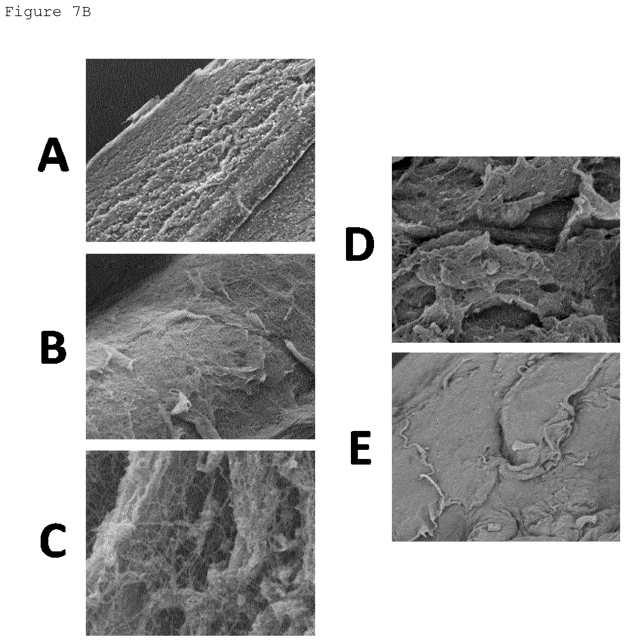

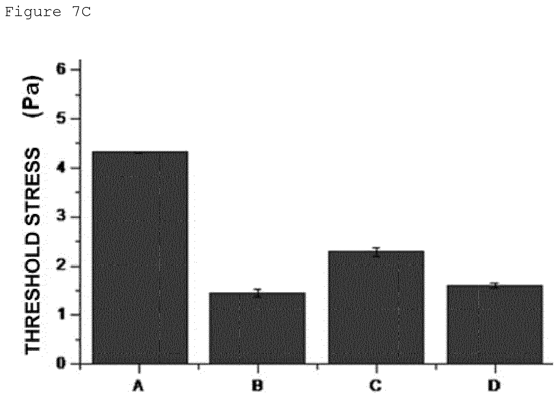

Figure 7 shows the evaluation of the artificial human oral mucosa products. A. Analysis by means of optical microscopy of the fibrin, agarose and collagen tissues (collagen at a final concentration of 2.8 g/L) stained with hematoxylin and eosin after 1, 2, 3 and 4 weeks of development in culture and of the stroma of the human oral mucosa used as control. B. Analysis by means of scanning electron microscopy of the fibrin-, agarose- and collagen-based artificial tissues with a final preferred collagen concentration of 2.8 g/L (A) in comparison with the artificial fibrin, agarose and collagen tissues with a final collagen concentration of 3.8 g/L (B), fibrin, agarose and collagen tissues with a final collagen concentration of 1.9 g/L (C), or collagen tissues at a final concentration of 5.6 g/L in the absence of fibrin and agarose (D) and of the stroma of the normal human oral mucosa used as control (E). C. Threshold stress of the artificial fibrin-, agarose- and collagen-based tissues with a final preferred collagen concentration of 2.8 g/L (A) in comparison with the artificial fibrin, agarose and collagen tissues with a final collagen concentration of 3.8 g/L (B), fibrin, agarose and collagen tissues with a final collagen concentration of 1.9 g/L (C), or collagen tissues at a final concentration of 5.6 g/L in the absence of fibrin and agarose (D).
Figure 8 shows the improvement of the threshold stress (in Pascal) of the nanostructured tissues with respect to the non-nanostructured tissues.
Figure 9 shows the viscous modulus G" of the samples subjected to nanostructuring and of the non-nanostructured samples (data in Pascal).
Figure 10 shows the elastic modulus G' of the nanostructured tissues and of the native non-nanostructured biomaterials.
Figure 11 shows the transparency of the tissues subjected to 0.1% fibrin-agarose nanostructuring, and of the non-nanostructured 0.1% fibrin-agarose biomaterials. The data are provided as the percentage of visible light spectrum transmission (from approximately 400 to 700 nm).

### Detailed Description of the Invention

Tissue engineering is one of the biotechnology areas which has undergone the most development in recent years due to its use for the *in vitro* production of tissues and organs for implant in patients needing these tissues. However, the limitations of artificial tissues existing until now make developing new techniques which allow obtaining artificial tissues which can be used in human clinical practice or for evaluating pharmacological and chemical products necessary.

### Method of the invention

The present invention provides an *in vitro* method for preparing an artificial tissue, the artificial tissue obtainable by said method and using this artificial tissue to partially or completely increase, restore or replace the functional activity of a damaged tissue or organ.

A first aspect of the invention relates to an *in vitro* method for preparing an artificial tissue (hereinafter, method of the invention) comprising:
a) adding a composition comprising fibrinogen to a sample of isolated cells,
b) adding an antifibrinolytic agent to the product resulting from step (a),
c) adding at least one coagulation factor, a source of calcium, thrombin, or any combination of the above to the product resulting from step (b),
d) adding a composition of a polysaccharide to the product resulting from step (c),
e) culturing isolated cells in or on the product resulting from step (d), and
f) inducing the nanostructuring of the product resulting from step (e), wherein said nanostructuring induction comprises the dehydration and/or mechanical compression of the product resulting from step (e), and wherein the polysaccharide of step (d) is agarose. In step (a) of the method of the invention a composition comprising fibrinogen is added to isolated cells, preferably, cells isolated from a mammal. Said cells can be obtained by means of different methods described in the state of the art which can depend on the particular related cell type. Some of these methods are, for example, but not limited to, biopsy, mechanical processing, enzymatic treatment (for example, but not limited to, treatment with trypsin or type I collagenase), centrifugation, erythrocyte lysis, filtration, culture in supports or media favoring the selective proliferation of said cell type or immunocytometry. Some of these methods are described in detail in the examples of this specification.

The cells of step (a) can be differentiated cells such as, for example, but not limited to, fibroblasts, keratocytes or smooth muscle cells, or undifferentiated cells with the capacity for differentiating into said cells such as, for example, adult stem cells.

In a preferred embodiment of the method of the invention, the cells of step (a) are fibroblasts or undifferentiated cells with fibroblast differentiating capacity. Fibroblasts can be obtained from any tissue or organ, however, the fibroblasts of step (a) preferably originate from the tissue or from the organ in which the artificial tissue is to be used as replacement. For example, when the method of the invention is used for preparing a skin replacing tissue or an artificial skin, the fibroblasts preferably originate from skin (dermal fibroblasts); when it is used for preparing a bladder replacing tissue or an artificial bladder, the fibroblasts preferably originate from bladder; when it is used for preparing a urethra replacing tissue or an artificial urethra the fibroblasts preferably originate from urethra; or when it is used for preparing an oral mucosa replacing tissue or an artificial oral mucosa the fibroblasts preferably originate from oral mucosa. Nevertheless, the fibroblasts can be obtained from any other tissue or organ, such as for example, the oral mucosa, the abdominal wall or any connective tissue. The fibroblasts obtained from oral mucosa can be used, for example, for preparing a skin replacing tissue or an artificial skin, a bladder replacing tissue or an artificial bladder, a urethra replacing tissue or an artificial urethra, or a cornea replacing tissue or an artificial cornea.

In another preferred embodiment of the method of the invention, the cells of step (a) are keratocytes or undifferentiated cells with keratocyte differentiating capacity. When the method of the invention is used, for example, for preparing a cornea replacing tissue or an artificial cornea, keratocytes from the corneal stroma are preferably used.

The possibility that all the components of the artificial tissue are of autologous origin allows the transplantation of said tissue to be performed without the need of suppressing the immune system of the transplanted subject. However, the components of the artificial tissue can also be of allogenic origin, i.e., they can originate from an individual different from the individual to whom the artificial tissue is to be transplanted. Even the species from which said components originate can be different; in which case the tissue origin is said to be xenogenic. This opens up the possibility that the artificial tissue is prepared in advance when it is needed urgently, although in this case it would be recommendable to suppress the immune system of the subject to whom the artificial tissue is transplanted.

Therefore, in a preferred embodiment, the cells of step (a) of the invention are of autologous origin. Nevertheless, the cells of step (a) can also be of allogenic or xenogenic origin.

By means of adding the different components described in steps (a)-(d) of the method of the invention to the cells of step (a), and after leaving the product resulting from step (e) to settle in a support, a matrix comprising fibrin, polysaccharide and in the corresponding case, the protein added in step (d2) if said step has been applied, is formed in which said cells are embedded and on which and/or in which the cells can grow. Preferably, the cells of step (a) grow inside said matrix.

The formation of a fibrin matrix happens through thrombin-induced fibrinogen polymerization. Fibrinogen is a high molecular weight protein which is present in blood plasma. Thrombin is a proteolytic enzyme causing the rupture of fibrinogen molecule into low molecular weight polypeptides and fibrin monomers. Said monomers polymerize into dimers and are subsequently bound to one another by means of covalent bonds through the action of factor XIII, previously activated by the thrombin, and in the presence of calcium ions.

The composition comprising fibrinogen of step (a) can be, for example, but not limited to, blood plasma. The composition of step (a) can also be prepared from a plasma derivative, such as, for example, but not limited to a fibrinogen cryoprecipitate or concentrate. In addition to fibrinogen, the composition of step (a) can contain other coagulation factors.

In a preferred embodiment, the fibrinogen concentration in the product resulting from step (c) is between 0.5 and 10 g/L, optionally between 1 and 10 g/L. In a more preferred embodiment, the concentration in the product resulting from step (d) is between 1 and 4 g/L, optionally between 2 and 4 g/L. Nevertheless, a greater or lower concentration could also be used.

In a preferred embodiment, the fibrinogen of the composition of step (a) or the composition comprising fibrinogen of step (a) is of autologous origin. Nevertheless, the fibrinogen of the composition of step (a) or the composition comprising fibrinogen of step (a) can also be of allogenic or xenogenic origin.

In a preferred embodiment of this first aspect of the invention, the fibrinogen containing composition of step (a) is blood plasma. In this case, fibrinogen polymerization can be induced by means of adding a source of calcium in step (c).

In a more preferred embodiment of this first aspect of the invention, the source of calcium of step (c) is a calcium salt such as, for example, but not limited to, calcium chloride, calcium gluconate or a combination of both. The concentration of the calcium salt must be sufficient to induce fibrinogen polymerization. In a more preferred embodiment, the calcium salt is calcium chloride. In a yet more preferred embodiment, the concentration of calcium chloride in the product resulting from step (e) is between 0.25 and 3 g/L, optionally between 0.5 and 4 g/L. Nevertheless, a greater or lower concentration could also be used.

As used herein, the term "coagulation factor" refers to a component, generally, a protein, present in the blood plasma which takes part in the chain reaction enabling coagulation. There are thirteen coagulation factors, numbered with Roman numerals: I: fibrinogen; II: prothrombin; III: tissue factor or thromboplastin; IV: calcium; V: proaccelerin; VI: inactive factor or zymogen; VII: proconvertin; VIII: antihemophilic factor A or von Willebrand factor; IX: antihemophilic factor B or Christmas factor; X: Stuart-Prower factor; XI: antihemophilic factor C; XII: Hageman factor; XIII: fibrin stabilizing factor; XIV: Fitzgerald; XV: Fletcher; XVI: platelets; and XVII: Somocurcio. Preferably, the other coagulation factor added in step (c) of the method of the present invention is factor XIII.

Fibrin polymer can be degraded by means of the process called fibrinolysis. During fibrinolysis, plasminogen is converted into active plasmin enzyme by the plasminogen tissue activator; the plasmin binds to the fibrin surface through its binding sites to cause the degradation of fibrin polymer. To prevent the fibrinolysis of the fibrin matrix, an antifibrinolytic agent is added in step (b) of the present invention such as, for example, but not limited to, epsilon aminocaproic acid, tranexamic acid or aprotinin.

Tranexamic acid is a synthetic product derived from the amino acid lysine with high affinity for the lysine binding sites of plasminogen; it blocks these sites and prevents the binding of activated plasminogen to the fibrin surface, exerting an antifibrinolytic effect. Tranexamic acid has the advantage, in comparison with other antifibrinolytic agents of animal origin, that it does not transmit diseases. Therefore, in a preferred embodiment, the antifibrinolytic agent is tranexamic acid. In a yet more preferred embodiment, the concentration of tranexamic acid in the product resulting from step (e) is between 0.5 and 2 g/L, preferably between 1 and 2 g/L. Nevertheless, a greater or lower concentration could also be used.

The fibrin matrices are very versatile, therefore they have been used for preparing different artificial tissues, however, the clinical use thereof has been limited due mainly due to their limited consistency, their difficult manipulation and their extreme fragility. For that reason, a polysaccharide is added in step (d) of the method of the invention. Said polysaccharide is generally used to provide resistance and consistency to the tissue, and it is convenient that the polysaccharide is soluble therein. It is disclosed that examples of polysaccharides which can be used in step (d) of the method of the present disclosure are, but without limitation, agar-agar, agarose, alginate, chitosan or carraghenates, or any combination of the above. The polysaccharide added in step (d) of the method of the invention is agarose.

Agarose is a polysaccharide formed by alpha and beta galactoses extracted from algae of the genera such as *Gellidium* or *Gracillaria.* Agarose, in comparison with other polysaccharides which can be used in step (d) of the present invention, has the advantage that it forms a matrix which is inert from the immunological view point. Therefore, in a preferred embodiment, the polysaccharide of step (d) of the method of the invention is agarose. There are different types of agarose which vary in their physical and chemical properties such as, for example, the gelling temperature, the gel resistance and/or porosity. Preferably, the agarose of step (d) of the method of the invention is an agarose with a low melting point, i.e., an agarose which repolymerizes and solidifies at a temperature, preferably, less than 65°C and, more preferably, less than 40°C; it can thus be used for preparing the tissue at very low temperatures, minimizing the probability of cell death. In a more preferred embodiment, the agarose used in step (d) of the method of the invention is agarose type VII. In a yet more preferred embodiment, the agarose, preferably, agarose type VII, in the product resulting from step (e) is at a concentration, advantageously between 0.1 and 6 g/L, optionally between 0.2 and 6 g/L, preferably between 0.15 and 3 g/L, optionally between 0.3 and 3 g/L and more preferably between 0.25 and 2 g/L, optionally between 0.5 and 2 g/L. Nevertheless, a greater or lower concentration could also be used.

In a preferred embodiment, the method of the invention comprises an additional step between step (b) and step (c) (step (b2)) in which a protein is added. Examples of proteins which can be used in step (b2) of the method of the present invention are, but without limitation, fibronectin, laminin, type VII collagen or entactin, or any combination of the above. In the tissues, these proteins naturally form part of the extracellular matrix of the connective tissue, therefore the cells embedded in an artificial tissue obtained by means of the method of the invention are at a micro-environment which is more similar to a physiological environment, improving the adhesion, the differentiation and/or the survival of said cells.

In a preferred embodiment, the protein which is added in step (b2) is fibronectin. Fibronectin is a glycoprotein present in the extracellular matrix (ECM) of most animal cellular tissues playing an important role in matrix cell adhesion. In a more preferred embodiment, the protein added between step (b) and step (c) of the method of the invention is fibronectin. The object of this addition is to favor the adhesion of the cells of step (e) to the product resulting from step (d). For example, when the method of the invention is used for preparing a cornea replacing tissue or an artificial cornea, adding fibronectin reduces the detachment of corneal epithelial cells added in step (e) which involves a significant advantage with respect to other methods described in the state of the art. In a yet more preferred embodiment, the concentration of fibronectin in the product resulting from step (d) is between 0.25 and 1g/L, optionally between 0.5 and 1 g/L. Nevertheless, a greater or lower concentration could also be used.

In a preferred embodiment, the method of the invention comprises an additional step (step d2) between steps (d) and (e) which comprises adding a composition comprising a protein to the product resulting from step (d). Examples of proteins which can be used in step (e) of the method of the present invention are, but without limitation, collagen, reticulin or elastin. Adding a protein between step (d) and step (e) produce tissues having a greater fibril density at the stroma level, a better viscoelastic behaviour and an increasing threshold stress. In a yet more preferred embodiment, the protein which is added in step (d2) is collagen.

Adding said proteins in step (d2) of the method of the invention also improves the physical properties (rheological, mechanical or biomechanical properties) of the artificial tissue obtained. The examples of this specification demonstrate that in the artificial tissues comprising fibrin, agarose and collagen, using increasing collagen concentration improves the viscoelastic behaviour, which is clearly shown by an increase of the threshold stress depending on the collagen concentration.

The main rheological properties of a solid or semisolid material are the viscosity and elasticity. Viscosity is the resistance of a fluid against tangential deformation, and it would be equivalent to consistency or rigidity. Elasticity is the mechanical property of certain materials of undergoing reversible deformations when they are subjected to the action of external forces, and to recover the original shape when these external forces cease. These parameters are analyzed by means of rheometry, physics technique using instruments called rheometers.

Threshold stress is the energy needed to cause an irreversible deformation in a solid or a fluid. Normally, all materials have an elastic region, in which the force applied causes a completely reversible deformation when the force ceases. If that force exceeds a limit (elastic modulus), the deformation becomes irreversible, entering into a plastic region. Finally, if the force exceeds the plastic modulus, the material breaks (yield point).

Collagen is a protein which is easily available in the nature and is biologically characterized by its low immunity and high tissue activity. Collagen forms the collagen fibres which are flexible but offer great traction resistance. The present invention demonstrates that the artificial fibrin, agarose and collagen tissues have a greater fibril density at the stroma level, a better viscoelastic behaviour and an increasing threshold stress as the collagen concentration increases, and higher than the artificial collagen tissues. Therefore, in a preferred embodiment the protein added in step (e) is collagen.

In a preferred embodiment, the collagen added in step (d2) is selected from the list comprising: type I collagen, type II collagen, type III collagen, type IV collagen, type V collagen, type VI collagen, type VII collagen, type VIII collagen, type IX collagen, type X collagen, type XI collagen, type XII collagen, type XIII collagen or any combination of the above. In a more preferred embodiment, the collagen added in step (e) is selected from the list comprising: type I collagen, type II collagen, type III collagen, type IV collagen, type V collagen, type IX collagen or any combination of the above. The selection of a particular type of collagen in step (e) of the method of the invention depends on the artificial tissue to be prepared and is made depending on the characteristics of each collagen which are known in the state of the art.

For example, the main function of type I collagen is to resist stretch and it is found abundantly in the dermis, bone, tendon and cornea. Therefore, the present invention demonstrates that adding type I collagen in step (e) renders excellent properties to the artificial tissue when, for example, but without limitation, a cornea replacing tissue or an artificial cornea is to be prepared. Therefore, in a preferred embodiment the collagen is type I collagen.

In a yet more preferred embodiment, the collagen, preferably, type I collagen, in the product resulting from step (d) is at a concentration advantageously between 0.5 and 5 g/L, preferably between 1.8 and 3.7 g/L, and more preferably between 2.5 and 3 g/L. Nevertheless, a greater or lower concentration could also be used.

In a particular embodiment, the collagen used is an atelocollagen, i.e., a collagen from which the terminal regions of non-helical structure called telopeptides have been removed. These telopeptides are the carriers of the main antigenic determinants of the collagen and can make the collagen insoluble. Atelocollagen is obtained, for example, by means of protease treatment with pepsin.

Depending on the fibrinogen concentrations used in step (a), the polysaccharide concentration used in step (d) and, in the case that a step (d2) is applied, on the collagen concentration used in step (d2), the artificial tissue resulting from step (e) can comprise variable concentrations of two/three components.

In a preferred embodiment, in the product resulting from step (e), the fibrinogen concentration is between 0.5 and 10 g/L, the agarose concentration, preferably, agarose type VII, is between 0.1 and 6 g/L. If a step (d2) has been included, the collagen concentration, preferably, type I collagen, is between 0.5 and 5 g/L.

In another preferred embodiment, in the product resulting from step (e), the fibrinogen concentration is between 0.5 and 10 g/L, the agarose concentration, preferably, agarose type VII, is between 0.15 and 3 g/L. If a step (d2) has been included, the collagen concentration, preferably, type I collagen, is between 0.5 and 5 g/L.

In another preferred embodiment, in the product resulting from step (e), the fibrinogen concentration is between 0.5 and 10 g/L, the agarose concentration, preferably, agarose type VII, is between 0.25 and 2 g/L. If a step (d2) has been included, the collagen concentration, preferably, type I collagen, is between 0.5 and 5 g/L.

In another preferred embodiment, in the product resulting from step (e), the fibrinogen concentration is between 0.5 and 10 g/L, the agarose concentration, preferably, agarose type VII, is between 0.1 and 6 g/L. If a step (d2) has been included, the collagen concentration, preferably, type I collagen, is between 1.8 and 3.7 g/L.

In another preferred embodiment, in the product resulting from step (e), the fibrinogen concentration is between 0.5 and 10 g/L, the agarose concentration, preferably, agarose type VII, is between 0.15 and 3 g/L. If a step (d2) has been included, the collagen concentration, preferably, type I collagen, is between 1.8 and 3.7 g/L.

In another preferred embodiment, in the product resulting from step (e), the fibrinogen concentration is between 0.5 and 10 g/L, the agarose concentration, preferably, agarose type VII, is between 0.25 and 2 g/L. If a step (d2) has been included, the collagen concentration, preferably, type I collagen, is between 1.8 and 3.7 g/L.

In another preferred embodiment, in the product resulting from step (e), the fibrinogen concentration is between 0.5 and 10 g/L, the agarose concentration, preferably, agarose type VII, is between 0.1 and 6 g/L. If a step (d2) has been included, the collagen concentration, preferably, type I collagen, is between 2.5 and 3 g/L.

In another preferred embodiment, in the product resulting from step (e), the fibrinogen concentration is between 0.5 and 10 g/L, the agarose concentration, preferably, agarose type VII, is between 0.15 and 3 g/L. If a step (d2) has been included, the collagen concentration, preferably, type I collagen, is between 2.5 and 3 g/L.

In another preferred embodiment, in the product resulting from step (e), the fibrinogen concentration is between 0.5 and 10 g/L, the agarose concentration, preferably, agarose type VII, is between 0.25 and 2 g/L. If a step (d2) has been included, the collagen concentration, preferably, type I collagen, is between 2.5 and 3 g/L.

In another preferred embodiment, in the product resulting from step (e), the fibrinogen concentration is between 1 and 4 g/L, the agarose concentration, preferably, agarose type VII, is between 0.1 and 6 g/L. If a step (d2) has been included, the collagen concentration, preferably, type I collagen, is between 0.5 and 5 g/L.

In another preferred embodiment, in the product resulting from step (e), the fibrinogen concentration is between 1 and 4 g/L, the agarose concentration, preferably, agarose type VII, is between 0.15 and 3 g/L. If a step (d2) has been included, the collagen concentration, preferably, type I collagen, is between 0.5 and 5 g/L.

In another preferred embodiment, in the product resulting from step (e), the fibrinogen concentration is between 1 and 4 g/L, the agarose concentration, preferably, agarose type VII, is between 0.25 and 2 g/L. If a step (d2) has been included, the collagen concentration, preferably, type I collagen, is between 0.5 and 5 g/L.

In another preferred embodiment, in the product resulting from step (e), the fibrinogen concentration is between 1 and 4 g/L, the agarose concentration, preferably, agarose type VII, is between 0.1 and 6 g/L. If a step (d2) has been included, the collagen concentration, preferably, type I collagen, is between 1.8 and 3.7 g/L.

In another preferred embodiment, in the product resulting from step (e), the fibrinogen concentration is between 1 and 4 g/L, the agarose concentration, preferably, agarose type VII, is between 0.15 and 3 g/L. If a step (d2) has been included, the collagen concentration, preferably, type I collagen, is between 1.8 and 3.7 g/L.

In another preferred embodiment, in the product resulting from step (e), the fibrinogen concentration is between 1 and 4 g/L, the agarose concentration, preferably, agarose type VII, is between 0.25 and 2 g/L. If a step (d2) has been included, the collagen concentration, preferably, type I collagen, is between 1.8 and 3.7 g/L.

In another preferred embodiment, in the product resulting from step (e), the fibrinogen concentration is between 1 and 4 g/L, the agarose concentration, preferably, agarose type VII, is between 0.1 and 6 g/L. If a step (d2) has been included, the collagen concentration, preferably, type I collagen, is between 2.5 and 3 g/L.

In another preferred embodiment, in the product resulting from step (e), the fibrinogen concentration is between 1 and 4 g/L, the agarose concentration, preferably, agarose type VII, is between 0.15 and 3 g/L. If a step (d2) has been included, the collagen concentration, preferably, type I collagen, is between 2.5 and 3 g/L.

In another preferred embodiment, in the product resulting from step (e), the fibrinogen concentration is between 1 and 4 g/L, the agarose concentration, preferably, agarose type VII, is between 0.25 and 2 g/L. If a step (d2) has been included, the collagen concentration, preferably, type I collagen, is between 2.5 and 3 g/L.

By means of adding the different components described in to the cells of step (a) once the steps (a)-(d) of the method of the invention are carried out, and after leaving the product resulting from step (e) to settle in a support, a matrix comprising fibrin, polysaccharide, in the case that step (d2) has been included, the added protein in said step, is formed, in which said cells are embedded and on which and/or in which the cells can grow. Preferably, the cells of step (a) grow inside said matrix.

Once the steps (a)-(d) of the method of the invention are carried out, the product resulting from step (d) is left to settle in a support so that the matrix comprising the fibrin, polysaccharide and, depending on whether a step (b2) has been carried out, the added protein in said step, is formed, which matrix has embedded therein the cells of step (a). Supports which can be used are, for example, but not limited to, culture tissue dishes or porous cell culture inserts. Preferably, said supports will be in sterile conditions.

Step (e) of the method of the invention consists of culturing isolated cells, preferably, cells isolated from a mammal, in or on the product resulting from step (e). Said cells can be obtained by means of different methods described in the state of the art and they can depend on the particular related cell type. Some of these methods are, for example, but not limited to, biopsy, mechanical processing, enzymatic treatment (for example, but not limited to, with trypsin or type I collagenase), centrifugation, erythrocyte lysis, filtration, culture in supports or media favoring the selective proliferation of said cell type or immunocytometry. Some of these methods are described in detail in the examples of this invention.

The cells of step (e) can be differentiated cells such as, for example, but not limited to, epithelial cells, or undifferentiated cells with the capacity for differentiating into said cells such as, for example, adult stem cells.

In a preferred embodiment, the differentiated cells of step (e) are epithelial cells, such as, for example, but not limited to, keratinocytes, oral mucosal epithelial cells, bladder epithelial cells, urethral epithelial cells, corneal epithelial cells or vascular endothelial cells.

Preferably, the epithelial cells of step (e) originates from the tissue or from the organ in which the artificial tissue is to be used as replacement. For example, when the method of the invention is used for preparing a skin replacing tissue or an artificial skin, the epithelial cells preferably originate from the skin epidermis, i.e., they are keratinocytes; when used for preparing a bladder replacing tissue or an artificial bladder, the epithelial cells preferably originate from the bladder epithelium or urothelium; when used for preparing a urethra replacing tissue or an artificial urethra the epithelial cells preferably originate from the urethral epithelium; when used for preparing a cornea replacing tissue or an artificial cornea preferably, the epithelial cells are corneal epithelial cells; when used for preparing an oral mucosa replacing tissue, preferably, the epithelial cells preferably originate from the oral mucosal epithelium.

However, the epithelial cells of step (e) can also be obtained from a tissue or organ different from the tissue or organ in which the artificial tissue is to be used as replacement. For example, when the method of the invention is used for preparing a skin replacing tissue or an artificial skin, a bladder replacing tissue or an artificial bladder, a urethra replacing tissue or an artificial urethra, or a cornea replacing tissue or an artificial cornea the epithelial cells of step (e) can be oral mucosal epithelial cells. Or, for example, when the method of the invention is used for preparing a bladder replacing tissue or an artificial bladder, or when is used for preparing a urethra replacing tissue or an artificial urethra, the epithelial cells can be keratinocytes.

One of the problems associated with artificial tissue generation in laboratory is the production of a significant number of differentiated cells, the use of stem cells, with capacity for differentiating into said cells is therefore commonly considered as an alternative source. "Stem cell" is understood as that having a high capacity for dividing and morphologically and functionally differentiating into different types of more specialized cells. During the differentiation process, an undifferentiated cell changes its phenotype and morphology to be converted into a differentiated cell with a specialized structure and function.

Stem cells can be classified according to their potential, i.e., their capacity for differentiating into different cell types, into: (a) totipotentials (not part of the invention): capable of differentiating both into embryonic tissue and extraembryonic tissue; (b) pluripotentials with capacity for differentiating into any of the tissues originating from the three embryonic layers (endoderm, mesoderm and ectoderm) ; (c) multipotentials: capable of differentiating into different cell types derived from one and the same embryonic layer (endoderm, mesoderm or ectoderm); and (d) unipotentials: capacity for forming a single cell lineage.

According to their origin, the stem cells have been divided into: (a) embryonic stem cells: from the inner mass cell of the blastula in the preimplantation embryo stage or from the gonadal crest, which are totipotentials (not part of the invention) or pluripotentials; and (b) adult stem cells: in the adult, the fetus and the umbilical cord, which are multipotentials or unipotentials. Mesenchymal stem cells which are distributed in the connective tissue of different organs, such as, for example, but not limited to, the bone marrow, peripheral blood, adipose tissue or umbilical cord are included among adult stem cells. In a preferred embodiment, the adult stem cells are adult stem cells from the bone marrow, the adipose tissue or the umbilical cord.

The umbilical cord is an interesting source of adult stem cells due to the fact that, unlike the adult stem cells obtained from other sources; (a) the method for obtaining them is not invasive or painful; and (b) their proliferative capacity and differentiation potential does not drop as a result of aging. Among the different sources of umbilical cord stem cells, the so-called umbilical cord Wharton's jelly stem cells stand out due to: (a) their great proliferation capacity and their speed of spreading in culture; and (b) the low expression of Class I major histocompatibility complex and the absence of expression of Class II major histocompatibility complex, making them good candidates for allogenic cell therapy.

Therefore, in another preferred embodiment, the cells of step (e) are umbilical cord Wharton's jelly stem cells. These cells express different mesenchymal cell characteristic markers on their surface such as, for example, SH2, SH3, CD10, CD13, CD29, CD44, CD54, CD73, CD90, CD105 or CD166, and they do not have hematopoietic lineage markers, such as for example, CD31, CD34, CD38, CD40 or CD45. The umbilical cord Wharton's jelly stem cells can be differentiated, for example, into chondroblasts, osteoblasts, adipocytes, neural precursors, cardiomyocytes, skeletal muscle cells, endothelial cells or hepatocytes.

Adult stem cells can be characterized by means of identifying surface and/or intracellular proteins, genes, and/or other markers indicative of their undifferentiated state, by means of different methods which are known in the state of the art such as, for example, but not limited to, immunocytometry, immunocytochemical analysis, northern blot analysis, RT-PCR, gene expression analysis in microarrays, proteomic studies or differential display analysis.

Stem cells can be induced to differentiate *in vitro* to produce cells expressing at least one or more typical characteristics of differentiated cells. Examples of differentiated cells which can be differentiated from the stem cells are, but not limited to, fibroblast, keratinocyte, urothelial cell, urethral epithelial cell, corneal epithelial cell, oral mucosal epithelial cell, chondroblast, osteoblast, adipocyte or neuron. In a preferred embodiment of the invention, the cell differentiated from the multipotent stem cell of the invention expresses one or more typical characteristics of a differentiated cell selected from the list comprising: fibroblast, keratinocyte, urothelial cell, urethral epithelial cell, corneal epithelial cell, oral mucosal epithelial cell, chondroblast, osteoblast, adipocyte or neuron.

The differentiated cells can be characterized by means of identifying the surface and/or intracellular proteins, genes, and/or other markers indicative of their differentiated state, by means of different methods which are known in the state of the art such as, for example, but not limited to, immunocytometry, immunocytochemical analysis, northern blot analysis, RT-PCR, gene expression analysis in microarrays, proteomic studies or differential display analysis.

In a preferred embodiment, the cells of step (e) of the invention are of autologous origin. Nevertheless, the cells of step (e) can also be of allogenic or xenogenic origin.

The cells of step (e) are capable of proliferating on the product resulting from step (d) and/or in the product resulting from step (d). Preferably, the cells of step (e) proliferate on the surface of the product resulting from step (d) .

The cells of step (e) are left to proliferate until reaching a suitable number typically at least 70% of confluence, advantageously, at least 80% of confluence, preferably, at least 90% of confluence, more preferably, at least 95% of confluence and, yet more preferably, at least 100% of confluence. While the cells are kept in culture, the culture medium where the cells are cultured can be partially or completely replaced with a new medium to replace exhautsed ingredients and to remove potentially harmful metabolites and catabolites.

An additional step may be necessary for the correct differentiation of some cell types. For example, in the case of oral mucosal epithelial cells, keratinocytes or corneal epithelial cells, it may be necessary to expose the epithelial surface to air to encourage the correct epithelium stratification and maturation keeping the matrix comprising the cells of step (a) submerged in the culture medium (air liquid technique).

Therefore, in a preferred embodiment, the method of the invention, in addition to steps (a)-(f) described above comprises an additional step in which the product resulting from step (e) is exposed to air. The method of the invention generally includes this step when it is used for obtaining an artificial tissue for replacing a natural tissue the epithelium of which is normally exposed to air contact such as, for example, but without limitation, the skin, the cornea, the oral mucosa, the urethra or the vagina. Preferably, this step is carried out when a skin replacing tissue or an artificial skin is prepared, or when a cornea replacing tissue or an artificial cornea is prepared, or when an oral mucosa replacing tissue or an artificial oral mucosa is prepared.

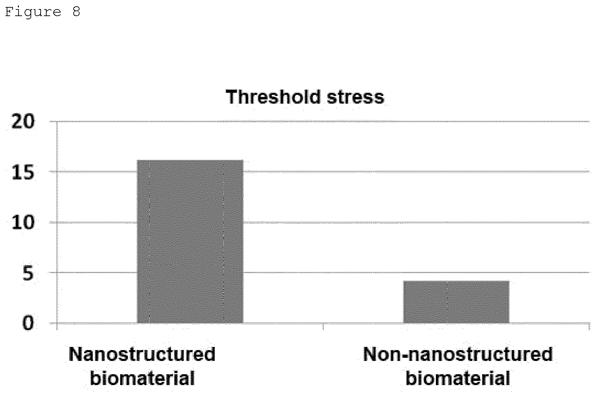

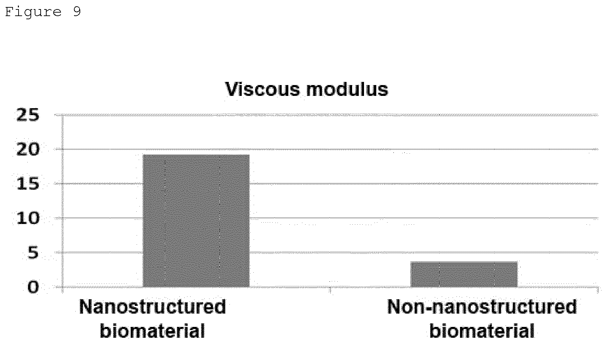

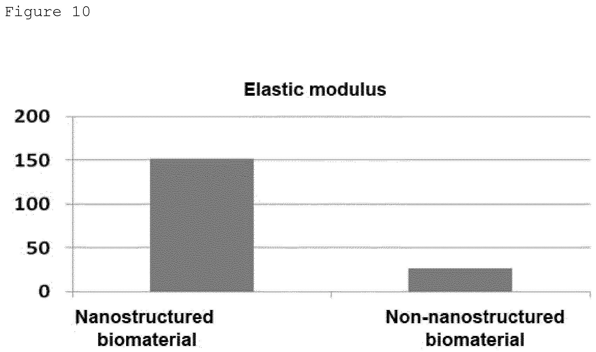

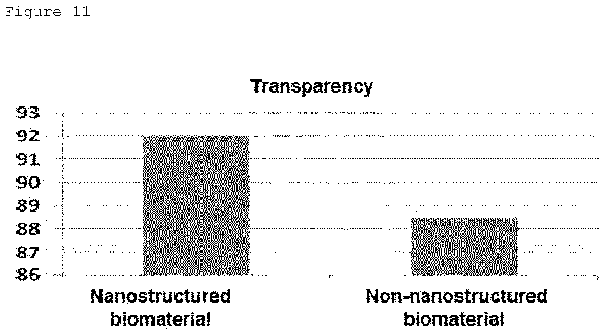

One of the most significant innovations of the method of the invention consists of the existence of a step (f) in which the nanostructuring of the product resulting from step (e) is induced. As used herein, the expression "nanostructuring" relates to a structural modification consisting of generating bonds having a size of less than one micron between the fibrin fibres and between fibrin fibres and agarose molecules. This nanostructuring process allows obtaining artificial tissues which unexpectedly show advantageous properties with respect to the non-structured biomaterials. Specifically, the biomaterials subjected to a nanostructuring process according to the present invention have (i) a significant improvement of tissue biomechanical properties, which allows manipulating the nanostructured tissue and it involves a substantial and unexpected improvement of the biomaterial rheological properties, characterized as a greater resistance (see Figure 8) and a greater elasticity (see Figures 9 and 10); (ii) a substantial improvement of the nanostructured tissue manipulability, which allowed its surgical manipulation, suturing to the recipient bed and implanting in test animals, (iii) a significant improvement of the transparency of the tissue subjected to nanostructuring (see Figure 11), which is not completely predictable since the nanostructured tissues are denser and have lower water content than the non nanostructured tissues and (iv) a better clinical result once implanted in related laboratory animals, on one hand, due to the greater clinical implant efficiency due to the suitable biomechanical properties of the biomaterial and, on the other hand, to the fact that the biomaterials subjected to nanostructuring have a greater fibre density per mm² and, therefore, a slower remodelization by the receiving organism. According to the invention, the nanostructuring induction of step (f) comprises the dehydration and/or mechanical compression of the product resulting from step (e). The objective of step (f) is to generate a structural modification between the fibrin fibres and the agarose molecules of the artificial tissue to achieve optimum consistency and elasticity levels, which are not obtained by means of other methods described in the state of the art. The end result is an irreversible modification of the fibres generating very favorable biomechanical qualities for surgical manipulation and clinical implant.

The term "dehydration" refers to a partial and/or total removal of interstitial fluid from the product resulting from step (e). For example, the amount of interstitial fluid removed from the product resulting from step (e) can be at least 50%, at least 60%, at least 70%, at least 80%, at least 90% or, at least 99% of the interstitial fluid originally contained in the product resulting from step (e).

The product resulting from step (e) can be dehydrated by means of any physical or chemical method. In a preferred embodiment, the dehydration of the product resulting from step (e) comprises a method selected from the list comprising: draining, evaporation, suction, capillary pressure, osmosis or electro-osmosis.

The interstitial fluid can be removed by means of inclining the product resulting from step (e); the interstitial fluid is then drained due to gravity and gradient effect.

The fluid can be removed by means of suction, for example, by applying vacuum by means of a mechanical pump to the surface where the product resulting from step (e) is.

The interstitial fluid can be removed by means of evaporation, for example, by incubating the product resulting from step (e) in conditions which encourage evaporation, for example, at a pressure less than the atmospheric pressure and/or at a temperature greater than the room temperature.

The interstitial fluid can also be removed using an osmotic agent with water absorbing tendency such as, for example, but not limited to, a hyperosmotic sodium chloride solution, separating the product resulting from step (e) from this solution by means of a semi-permeable membrane, a sponge or another drying material.

In a preferred embodiment, the interstitial fluid can be removed by means of capillary pressure, for example, by means of applying an absorbent material to the product resulting from step (e). Some examples of absorbent material which could be used in step (f) of the invention are, but not limited to, filter paper, 3M paper from the company Whatman, cellulose fibre, or absorbent fabric. The absorbent material will preferably be sterilized.

The time required for dehydrating will depend on the method or methods used, and it can be easily determined by a person skilled in the art. The suitability of the artificial tissue obtained by means of applying a specific dehydrating method for a specific time period can be confirmed by means of different evaluation methods known in the state of the art such as, for example, but not limited to, those described in the examples of this specification.

The interstitial fluid can also be removed by means of the mechanical compression of the product resulting from step (e). The mechanical compression of the product resulting from step (e) can also render a specific desired form to the product resulting from step (e).

The product resulting from step (e) can be compressed by means of any method described in the state of the art. A "static" compression method can be used, wherein the product resulting from step (e) remains stationary such as, for example, but not limited to, the application of a static load (for example, a deadweight), a hydraulic element or a cam. A "dynamic" compression method can also be used, wherein the product resulting from step (e) moves during the compression such as, for example, by means of the application of one or more rollers or by means of extrusion through a constricting hole.

The product resulting from step (e) can be mechanically compressed by means of extrusion, for example, by means of passing the product resulting from step (e) through a hole constricting it, for example, a conical chamber. The conical chamber can have porous walls, such that it would allow the removal of interstitial fluid from the product resulting from step (e) while it passes through the same.

The product resulting from step (e) can be compressed by means of centrifuging the product resulting from step (e). For example, the product resulting from step (e) can be placed on a tube with the porous bottom, such that, in addition to the mechanical compression, the removal of interstitial fluid from the product resulting from step (e) would occur.

The product resulting from step (e) can be compressed by means of applying a balloon therein to compress the product resulting from step (e) against a solid surface. The solid surface can, for example, form a tube surrounding the product resulting from step (e) allowing the formation of an artificial tubular tissue.

In a preferred embodiment, the compression of the product resulting from step (e) comprises applying a weight on top of the product resulting from step (e), such that a mechanical action of pressure is exerted on the tissue. It is obvious that the greater the weight the lesser the time needed for obtaining an artificial tissue with the suitable characteristics will be. The weight used for compressing can have a flat surface or can be placed on a material having a flat surface, for example, plastic, ceramic, metal or wood.

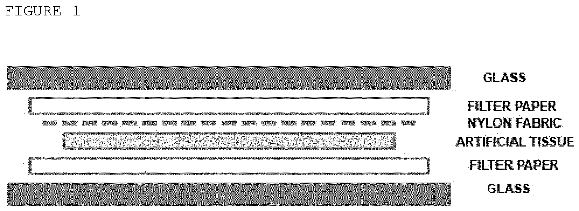

Figure 1 of the present specification shows a nonlimiting diagram of how the nanostructuring of the product resulting from step (e) can be performed by means of the dehydration and compression thereof. It can observed in said diagram how the nanostructuring can be obtained by locating the product resulting from step (e) between two sterile filter papers, and placing thereon a weight of approximately 250 g (equivalent to approximately 2,000 N/m²) on a sterile flat glass surface for approximately 10 minutes; a porous material can be arranged between the tissue and the filter paper on which the weight is placed to prevent the product resulting from step (e) from adhering to the filter paper. The material used to prevent the adherence must be porous to allow the exit of water from the tissue towards the dehydrating agent: said porous material used to prevent the adherence can be, for example, but not limited to, a nylon, glass, ceramic, perforated metal or polycarbonate membrane.

In a preferred embodiment, the compression of the product resulting from step (e) comprises applying a pressure thereon. The magnitude of the pressure is preferably between 1,000 and 5,000 N/m2, more preferably between 1,500 and 2,500 N/m² and, yet more preferably, of approximately 2,000 N/m². Said pressure can be applied manually, automatically or semiautomatically. The time needed to exert the pressure depends on the magnitude of the pressure applied and it can be easily determined by the person skilled in the art. It is obvious that the greater the pressure the lesser the time needed for obtaining an artificial tissue with the suitable characteristics will be. The suitability of the artificial tissue obtained by means of applying a specific magnitude of pressure for a specific time period can be confirmed by means of different evaluation methods known in the state of the art such as, for example, but not limited to, those described in the examples of this specification.

One or more methods can be sequentially or simultaneously used to induce the nanostructuring of the product resulting from step (e). The time required for nanostructuring may be less than 12 hours, less than 6 hours, less than 3 hours, less than 1 hour, less than 30 minutes, less than 10 minutes, less than 2 minutes or less than 1 minute. The time required for nanostructuring will depend on the method or methods used, and it can be easily determined by the person skilled in the art. The suitability of the artificial tissue obtained by means of applying a specific method for a specific time period can be confirmed by means of different evaluation methods known in the state of the art such as, for example, but not limited to, those described in the examples of this specification.

### Artificial tissue of the invention

A second aspect of the present invention relates to an artificial tissue obtainable by the method of the invention described above (hereinafter, artificial tissue of the invention).

In a preferred embodiment of this second aspect of the invention, the artificial tissue of the invention is a skin replacing tissue or an artificial skin.

In another preferred embodiment of this second aspect of the invention, the artificial tissue is a bladder replacing tissue or an artificial bladder.

In another preferred embodiment of this second aspect of the invention, the artificial tissue of the invention is a urethra replacing tissue or an artificial urethra.

In another preferred embodiment of this second aspect of the invention, the artificial tissue of the invention is a cornea replacing tissue or an artificial cornea.

In another preferred embodiment of this second aspect of the invention, the artificial tissue of the invention is a mucosa replacing tissue or an artificial mucosa.

The artificial tissue obtainable by the method of the invention can be cut into the desired size and/or can be provided in a suitable conformation for use.

Before use, the suitability of the artificial tissue of the invention for performing its function can be evaluated, for example, but not limited to, by means of any of the methods described in the examples of the present description.

### Uses of the artificial tissue of the invention in pharmacological or chemical product evaluation

The drugs and chemical products must be evaluated before their administration into test animals. To this respect, there are several reports and directives approved by the European Union which aim to restrict or even prohibit animal testing in the sector of cosmetic products (Directive 76/768/EEC of the European Council relating to the approximation of Member States' laws on cosmetic products), and the complete ban is expected to be in force in the next few years. The European Union supports all the measures the main objective of which is the wellbeing of the animals used for testing purposes and for achieving scientific replacement methods to reduce the number of animals used for testing to the minimum (Decision 1999/575/EEC of the Council, dated 23 March 1998, relating to the conclusion by the Community of the European Convention for the protection of vertebrate animals used for experimental and other scientific purposes - Official Record L 222 of 24.08.1999).

Therefore, a third aspect of the invention relates to the use of the artificial tissue of the invention for evaluating a pharmacological and/or chemical product.

### Therapeutic uses of the artificial tissues of the invention

An infectious, inflammatory, genetic or degenerative disease, physical or chemical damage, or blood flow interruption, can cause cell loss from a tissue or organ. This cell loss would lead to an alteration of the normal function of said tissue or organ; and consequently lead to the development of diseases or physical consequences reducing the person's quality of life. Therefore, attempting to regenerate or and reestablish the normal function of said tissues or organs is important. The damaged tissue or organ can be replaced by a new tissue or organ which has been produced in the laboratory by means of tissue engineering techniques. The objective of tissue engineering is to construct artificial biological tissues and to use them for medical purposes to restore, replace or increase the functional activities of diseased tissues and organs. The therapeutic use of techniques of this type is virtually unlimited with applications in all fields. The use of tissue engineering techniques allows reducing the waiting lists for tissues and organs, with the consequent reduction the disease morbidity-mortality in recipient. As a consequence, it also logically reduces the morbidity-mortality in organ donors. In addition, there are many advantages associated with the use of autologous cells or tissues in tissue engineering, which include: (a) a significant reduction of the number of infections from the donor to the recipient by infectious agents; and (b) the absence of host immune graft rejection, therefore the patient does not need to undergo immunosuppressing treatment, side effects and problems associated with immunodepression being prevented.

Therefore, a fourth aspect of the invention relates to the artificial tissue of the invention for use in partially or completely increasing, restoring or replacing the functional activity of a diseased or damaged tissue or organ.

The artificial tissue of the invention can be used to partially or completely increase, restore or replace the functional activity of any diseased or damaged tissue or organ of a living organism. The tissue or organ can be internal tissue or organ such as, for example, but not limited to, the urethra or the bladder, or external tissue or organ such as, for example, but not limited to, the cornea or the skin. In a preferred embodiment, the damaged tissue or the organ are selected from the list comprising: skin, bladder, urethra, cornea, mucosa, conjunctiva, abdominal wall, conjunctiva, eardrum, pharynx, larynx, intestine, peritoneum, ligament, tendon, bone, meninx or vagina. The tissue or the organ can be diseased or damaged as a result of a dysfunction, an injury or a disease, for example, but not limited to, an infectious disease, an inflammatory disease, a genetic disease or a degenerative disease; physical damage such as a traumatism or a surgical intervention, a chemical damage or blood flow interruption.

A preferred embodiment of this fifth aspect relates to the artificial tissue of the invention for use in partially or completely increasing, restoring or replacing the functional activity of a skin. A more preferred embodiment relates to the artificial tissue of the invention for use in partially or completely increasing, restoring or replacing the functional activity of a diseased or damaged skin as a result of a dysfunction, an injury or a disease selected from the list comprising: a wound, an ulcer, a burn, a benign or malignant neoplasm, an infection, a bruise, a traumatism, a caustication or a congenital malformation.

A preferred embodiment of this fifth aspect relates to the artificial tissue of the invention for use in partially or completely increasing, restoring or replacing the functional activity of a bladder. A more preferred embodiment relates to the artificial tissue of the invention for use in partially or completely increasing, restoring or replacing the functional activity of a diseased or damaged bladder as a result of a dysfunction, an injury or a disease selected from the list comprising: a benign or malignant neoplasm, an infection, a traumatism, a congenital malformation (such as for example, but not limited to, bladder exstrophy, contracted bladder or cloaca exstrophy), a neurogenic bladder, urinary incontinence, a bladder dysfunction, an infection or a bladder lithiasis.

A preferred embodiment of this fifth aspect relates to the artificial tissue of the invention for use in partially or completely increasing, restoring or replacing the functional activity of a urethra. A more preferred embodiment relates to the artificial tissue of the invention for use in partially or completely increasing, restoring or replacing the functional activity of a diseased or damaged urethra as a result of a dysfunction, an injury or a disease selected from the list comprising: a benign or malignant neoplasm, an infection, a traumatism, a congenital malformation (such as for example, but not limited to, hypospadias or epispadias) or a stenosis.

A preferred embodiment of this fifth aspect relates to the artificial tissue of the invention for use in partially or completely increasing, restoring or replacing the functional activity of a cornea. A more preferred embodiment relates to the artificial tissue of the invention for use in partially or completely increasing, restoring or replacing the functional activity of a diseased or damaged cornea as a result of a dysfunction, an injury or a disease selected from the list comprising: a corneal ulcer, a keratoconus, a keratoglobus, a descemetocele, a traumatism, a caustication, a limbic deficiency, an atrophic keratitis, a corneal dystrophy, a primary or secondary keratopathy, an infection, a leukoma, a bullous keratopathy, a corneal endotelial failure or a benign or malignant neoplasm.

A preferred embodiment of this fifth aspect relates to the artificial tissue of the invention for use in preparing a medicament to partially or completely increase, restore or replace the functional activity of a mucosa, preferably of an oral mucosa. A yet more preferred embodiment relates to the artificial tissue of the invention for use in partially or completely increasing, restoring or replacing the functional activity of a diseased or damaged oral mucosa as a result of a dysfunction, an injury or a disease selected from the list comprising: a wound, an ulcer, a burn, a benign or malignant neoplasm, an infection, a bruise, a traumatism, a caustication, a congenital malformation, a substance loss or a periodontal disease. In a preferred embodiment, the tissue used to partially or completely increase, restore or replace the functional activity of a mucosa is a tissue which has been subjected a to step (d2) of adding a protein between step (d) and step (e). In a yet more preferred embodiment, said step is carried out by means of adding a composition comprising collagen to the material obtained in step (d) as described in detail above.

A fifth aspect of the present invention relates to the artificial tissue of the invention for use in preparing a medicament.

Said medicament is a medicament for somatic cell therapy. "Somatic cell therapy" is understood as the use of living, autologous, allogenic or xenogenic somatic cells, the biological characteristic of which have been substantially altered as a result of their manipulation for obtaining a therapeutic, diagnostic or preventive effect through metabolic, pharmacological or immunological means. Among the medicaments for somatic cell therapy are, for example, but not limited to: cells manipulated to modify their immunological, metabolic or other type of functional properties in qualitative and quantitative aspects; sorted, selected and manipulated cells which are subsequently subjected to a manufacturing process for the purpose of obtaining the end product; cells manipulated and combined with non-cellular components (for example, biological or inert matrices or medical devices) performing the principle intended action in the finished product; autologous cell derivatives expressed ex *vivo (in vitro)* under specific culture conditions; cells which are genetically modified or are subjected to another type of manipulation to express homologous or non-homologous functional properties not expressed before.

A fifth aspect of the present invention relates to the artificial tissue of the invention for use in preparing a medicament to partially or completely increase, restore or replace the functional activity of a tissue or organ. In a preferred embodiment, the damaged tissue or organ is selected from the list comprising: skin, bladder, urethra, cornea, mucosa, conjunctiva, abdominal wall, conjunctiva, eardrum, pharynx, larynx, intestine, peritoneum, ligament, tendon, bone, meninx or vagina.

A preferred embodiment of this fifth aspect relates to the artificial tissue of the invention for use in preparing a medicament to partially or completely increase, restore or replace the functional activity of a diseased or damaged tissue or organ as a result of an infectious disease, inflammatory disease, genetic disease or degenerative disease, physical or chemical damage or blood flow interruption.

A more preferred embodiment of this fifth aspect relates to the artificial tissue of the invention for use in preparing a medicament to partially or completely increase, restore or replace the functional activity of skin. A yet more preferred embodiment relates to the artificial tissue of the invention for use in preparing a medicament to partially or completely increase, restore or replace the functional activity of a diseased or damaged skin as a result of a dysfunction, an injury or a disease selected from the list comprising: a wound, an ulcer, a burn, a benign or malignant neoplasm, an infection, a bruise, a traumatism, a caustication or a congenital malformation.

A more preferred embodiment of this fifth aspect relates to the artificial tissue of the invention for use in preparing a medicament to partially or completely increase, restore or replace the functional activity of a bladder. A yet more preferred embodiment of this fifth aspect relates to the artificial tissue of the invention for use in preparing a medicament to partially or completely increase, restore or replace the functional activity of a diseased or damaged bladder as a result of a dysfunction, an injury or a disease selected from the list comprising: a benign or malignant neoplasm, an infection, a traumatism, a congenital malformation (such as for example, but not limited to, bladder exstrophy, contracted bladder or cloaca exstrophy), a neurogenic bladder, an urinary incontinence, a bladder dysfunction, an infection or a bladder lithiasis.

A more preferred embodiment of this fifth aspect relates to the artificial tissue of the invention for use in preparing a medicament to partially or completely increase, restore or replace the functional activity of a urethra. A yet more preferred embodiment of this fifth aspect relates to the artificial tissue of the invention for use in preparing a medicament to partially or completely increase, restore or replace the functional activity of a diseased or damaged urethra as a result of a dysfunction, an injury or a disease selected from the list comprising: a benign or malignant neoplasm, an infection, a traumatism, a congenital malformation (such as for example, but not limited to, hypospadias or epispadias) or a stenosis.

A more preferred embodiment of this fifth aspect relates to the artificial tissue of the invention for use in preparing a medicament to partially or completely increase, restore or replace the functional activity of a cornea. A yet more preferred embodiment of this fifth aspect relates to the artificial tissue of the invention for use in preparing a medicament to partially or completely increase, restore or replace the functional activity of a diseased or damaged cornea as a result of a dysfunction, an injury or a disease selected from the list comprising: a corneal ulcer, a keratoconus, a keratoglobus, a descemetocele, a traumatism, a caustication, a limbic deficiency, an atrophic keratitis, a corneal dystrophy, a primary or secondary keratopathy, an infection, a leukoma, a bullous keratopathy, a corneal endothelial failure or a benign or malignant neoplasm.

A more preferred embodiment of this fifth aspect relates to the artificial tissue of the invention for use in preparing a medicament to partially or completely increase, restore or replace the functional activity of a mucosa, preferably an oral mucosa. A yet more preferred embodiment relates to the artificial tissue of the invention for use in preparing a medicament to partially or completely increase, restore or replace the functional activity of a diseased or damaged oral mucosa as a result of a dysfunction, an injury or a disease selected from the list comprising: a wound, an ulcer, a burn, a benign or malignant neoplasm, an infection, a bruise, a traumatism, a caustication, a congenital malformation, a substance loss or a periodontal disease. In a preferred embodiment, the tissue used for preparing a medicament to partially or completely increase, restore or replace the functional activity of a mucosa is a tissue which has been subjected to a step (d2) of adding a protein between step (d) and step (e). In a yet more preferred embodiment, said step is carried out by means of adding a composition comprising collagen to the material obtained in step (d) as described in detail above.

A sixth aspect of the invention relates to a pharmaceutical composition comprising the artificial tissue of the invention.

A preferred embodiment of this sixth aspect of the invention relates to a pharmaceutical composition comprising the artificial tissue of the invention for use in somatic cell therapy.

A more preferred embodiment of this sixth aspect of the invention relates to a pharmaceutical composition comprising the artificial tissue of the invention to partially or completely increase, restore or replace the functional activity of a tissue or organ.

A preferred embodiment of this sixth aspect of the invention relates to a pharmaceutical composition comprising the artificial tissue of the invention to partially or completely increase, restore or replace the functional activity of a diseased or damaged tissue or organ as a result of an infectious disease, inflammatory disease, genetic disease or degenerative disease, physical or chemical damage or blood flow interruption.

A more preferred embodiment of this sixth aspect of the invention relates to a pharmaceutical composition comprising the artificial tissue of the invention to partially or completely increase, restore or replace the functional activity of a skin. A yet more preferred embodiment relates to a pharmaceutical composition comprising the artificial tissue of the invention to partially or completely increase, restore or replace the functional activity of a diseased or damaged skin as a result of a dysfunction, an injury or a disease selected from the list comprising: a wound, an ulcer, a burn, a benign or malignant neoplasm, an infection, a bruise, a traumatism, a caustication or a congenital malformation.

A more preferred embodiment of this sixth aspect of the invention relates to a pharmaceutical composition comprising the artificial tissue of the invention to partially or completely increase, restore or replace the functional activity of a bladder. A yet more preferred embodiment relates to a pharmaceutical composition comprising the artificial tissue of the invention to partially or completely increase, restore or replace the functional activity of a diseased or damaged bladder as a result of a dysfunction, an injury or a disease selected from the list comprising: a benign or malignant neoplasm, an infection, a traumatism, a congenital malformation (such as for example, but not limited to, bladder exstrophy, contracted bladder or cloaca exstrophy), a neurogenic bladder, an urinary incontinence, a bladder dysfunction, an infection or a bladder lithiasis.

A more preferred embodiment of this sixth aspect of the invention relates to a pharmaceutical composition comprising the artificial tissue of the invention to partially or completely increase, restore or replace the functional activity of a urethra. A yet more preferred embodiment relates to a pharmaceutical composition comprising the artificial tissue of the invention to partially or completely increase, restore or replace the functional activity of a diseased or damaged urethra as a result of a dysfunction, an injury or a disease selected from the list comprising: a benign or malignant neoplasm, an infection, a traumatism, a congenital malformation (such as for example, but not limited to, hypospadias or epispadias) or a stenosis.

A more preferred embodiment of this sixth aspect of the invention relates to a pharmaceutical composition comprising the artificial tissue of the invention to partially or completely increase, restore or replace the functional activity of a cornea. A yet more preferred embodiment relates to a pharmaceutical composition comprising the artificial tissue of the invention to partially or completely increase, restore or replace the functional activity of a diseased or damaged cornea as a result of a dysfunction, an injury or a disease selected from the list comprising: a corneal ulcer, a keratoconus, a keratoglobus, a descemetocele, a traumatism, a caustication, a limbic deficiency, an atrophic keratitis, a corneal dystrophy, a primary or secondary keratopathy, an infection, a leukoma, a bullous keratopathy, a corneal endothelial failure or a benign or malignant neoplasm.

A more preferred embodiment of this fifth aspect relates to a pharmaceutical composition comprising the artificial tissue of the invention for preparing a medicament to partially or completely increase, restore or replace the functional activity of a mucosa, preferably an oral mucosa. A yet more preferred embodiment relates to pharmaceutical composition comprising the artificial tissue of the invention to partially or completely increase, restore or replace the functional activity of a diseased or damaged oral mucosa as a result of a dysfunction, an injury or a disease selected from the list comprising: a wound, an ulcer, a burn, a benign or malignant neoplasm, an infection, a bruise, a traumatism, a caustication, a congenital malformation, a substance loss or a periodontal disease. In a form the pharmaceutical composition comprising the artificial tissue of the invention for preparing a medicament to partially or completely increase, restore or replace the functional activity of a mucosa or of an oral mucosa is a tissue which has been subjected to step (d2) of adding a protein between step (d) and step (e). In a yet more preferred embodiment, said step is carried out by means of adding a composition comprising collagen to the material obtained in step (d) as described in detail above.

In a preferred embodiment of this aspect of the invention, the pharmaceutical composition comprises the artificial tissue of the invention and also a pharmaceutically acceptable carrier. In another preferred embodiment of this aspect of the invention, the pharmaceutical composition comprises the artificial tissue of the invention and also another active ingredient. In a preferred embodiment of this aspect of the invention, the pharmaceutical composition comprises the artificial tissue of the invention and also another active ingredient together with a pharmaceutically acceptable carrier.

As used herein, the term "active ingredient", "active substance", "pharmaceutically active substance", "active ingredient" or "pharmaceutically active ingredient" means any component which potentially provides a pharmacological activity or another different effect in diagnosing, curing, mitigating, treating, or preventing a disease, or which affects the structure or function of the human body or body of other animals.

The pharmaceutical compositions of the present invention can be used in a treatment method in an isolated manner or together with other pharmaceutical compounds.

Along the description and claims, the word "comprises" and variants thereof do not intend to exclude other technical features, supplements, components or steps. For persons skilled in the art, other objects, advantages and features of the invention will be understood in part from the description and in part from the practice of the invention. The following examples and drawings are provided by way of illustration and they are not meant to limit the present invention.

### Examples

The following specific examples provided in this patent document serve to illustrate the nature of the present invention. These examples are included only for illustrative purposes and must not be interpreted as limiting to the invention which claimed herein. Therefore, the examples described below illustrate the invention without limiting the field of application thereof.

### Example 1. Protocol for preparing an artificial human skin product

### A.-Obtaining human skin samples.

Full thickness skin samples obtained from donors under local and locoregional anesthesia are used. Once the sample is sterilely obtained, the subcutaneous fatty tissue will be removed with the aid of scissors until exposing the dermis layer. The removed tissues will then be immediately introduced in a sterile transport medium made up of Dulbecco's Modified Eagle Medium (DMEM) supplemented with antibiotics (500 U/ml of penicillin G and 500 µg/ml of streptomycin) and antimycotic agents (1.25 µg/ml of amphotericin B) to prevent a possible sample contamination.

### B.- Generating primary fibroblast and keratinocyte cultures.

After the transport period, all the samples must be washed two times in a sterile PBS solution with penicillin, streptomycin and amphotericin B (500 U/ml, 500 µg/ml and 1.25 µg/ml, respectively) to remove all the blood, fibrin, fat or foreign material residues which may adhere to the samples.

First, to separate the dermis from the epidermis the samples are incubated at 37°C in a sterile solution with dispase II at 2 mg/ml in PBS. The basal membrane on which the epithelium anchors to the dermis is thus disintegrated, therefore after this, on one hand, the epithelium and on the other, the dermis will mechanically separated.

Once separated, the epithelium corresponding to the epidermis is fragmented with scissors until obtaining small fragments to subsequently incubate them in a trypsin-EDTA solution. This epithelium is transferred to a flask with a previously sterilized magnetic stirrer with the aid of a trypsin-EDTA soaked pipette. 2.5 ml of trypsin-EDTA is added to the flask with a stirrer and incubated at 37°C for 10 minutes to enzymatically separate the keratinocytes from the epidermis. After this time the trypsin which contained the separated keratinocytes is collected in a 50 ml conical tube with the aid of a pipette, trying not to drag the epithelium fragments there it. It is neutralized with an equal amount of culture medium supplemented with 10% fetal bovine serum and centrifuged for 10 minutes at 1000 rpm. The resulting pellet containing a large amount of separated keratinocytes is resuspended in 2-3 ml of keratinocyte culture medium which preferably favors epithelial cell growth over fibroblasts. This medium is made up of 3 parts of glucose rich DMEM medium and one part of Ham F-12 medium, all of this supplemented with 10% fetal bovine serum, antibiotics-antimycotic agents (100 U/ml of penicillin G, 100 µg/ml of streptomycin and 0.25 µg/ml of amphotericin B), 24 µg/ml of adenine and different growth factors: 0.4 µg/ml of hydrocortisone, 5 µg/ml of insulin, 10 ng/ml of epidermal growth factor (EGF), 1.3 ng/ml of triiodothyronine and 8 ng/ml of cholera toxin.

To digest the extracellular matrix of the skin's dermis and separate the stromal fibroblasts included in said matrix, the samples must be incubated at 37°C in a 2 mg/ml sterile solution of Clostridium hystoliticum type I collagenase in fetal bovine serum-free DMEM culture medium for 6 hours. This solution is capable of digesting the dermis collagen and freeing the stromal fibroblasts. To obtain primary fibroblast cultures, the digestion solution containing the digested stromal cells of the dermis must be centrifuged at 1,000 rpm for 10 minutes and the cell pellet corresponding to the fibroblasts is cultured in culture flasks having 15 cm² of surface area. Glucose enriched DMEM supplemented with antibiotics and antimycotic agents (100 U/ml of penicillin G, 100 µg/ml of streptomycin and 0.25 µg/ml of amphotericin B) and 10% fetal bovine serum (FBS) is used as the culture medium. This basic culture medium is called fibroblast medium.

In every case the cells will be incubated at 37°C with a 5% carbon dioxide in standard cell culture conditions. The culture media are renewed every three days.

### C.- Subculturing the cells originating from primary skin cultures.

Once the cell confluence is reached, the different fibroblast or keratinocyte cell cultures must be washed with sterile PBS and incubated in 1-3 ml of a solution of 0.5 g/l trypsin and 0.2 g/l EDTA at 37°C for 10 minutes. Therefore the cell adhesion mechanisms are disintegrated and separated cells not adhered to the surface of the keratinocyte and dermis culture flask are obtained.

For the case of dermis, once the cells detached from the surface of the culture flasks, the trypsin used is inactivated by means of adding 10 ml of fibroblast culture medium. The presence of abundant serum proteins is capable of inactivating the proteolytic action of trypsin. Keratinocyte culture medium is used in the keratinocytes.

The keratinocyte and fibroblast solutions in which the detached cells are found, are subsequently centrifuged at 1000 rpm for 10 minutes to obtain a cell pellet or cluster with the cells of interest, the supernatant with trypsin being discarded. The cell pellet is carefully resuspended in 5 ml of culture medium and these cells are cultured in culture flasks having 15, 25 or 75 cm² of surface area.

The keratinocyte cultures are normally expanded up to approximately five times in new culture flasks.

In every case and to assure an adequate cell viability, skin replacements was prepared out using cells corresponding to the four first subcultures.

### D.- Constructing fibrin- and agarose-based artificial human skin products by means of tissue engineering.

1- Generating dermis replacements with fibroblasts immersed therein using extracellular fibrin and agarose matrices. These dermal replacements will be directly generated on porous inserts of 0.4 µm in diameter to allow the passage of nutrients but not of the cells themselves. 10 ml of dermal replacement will be prepared in the following manner:
   - obtaining 7.6 ml of human blood plasma from donations (possibility of autologous origin).
   - adding 150,000 human skin fibroblasts which were previously cultured and resuspended in 750 µl of DMEM culture medium.
   - adding 150 µl of tranexamic acid to prevent the fibrinolysis of fibrin gel.
   - adding 1 ml of 1 % ClCa₂ to induce the coagulation reaction and generate a fibrin fibre network.
   - quickly adding 0.5 ml of 2% agarose type VII dissolved in PBS and heating until reaching the melting point and gently mixing by means of stirring. The final agarose concentration in the mixture will be 0.1%. The agarose concentration range allowing viable dermal products ranges between 0.025% and 0.3% and it must be established in relation to the patient object of use.
   - aliquoting as soon as possible in the porous cell culture inserts.
   - leaving it to polymerize at rest for at least 30 min.
2- Developing an epithelium layer (epidermis) on the surface of the dermal replacement by means of subculturing the keratinocytes on the dermal replacement. Covering with culture medium specific for keratinocytes.
3- Maintaining it in an incubator at 37°C with 5% CO2 at humid environment following standard cell culture protocols. The culture media are renewed every 3 days until the epithelial cells reach confluence on the surface of the dermal replacement (about one week).
4- Exposing the epithelial surface to air (air-liquid technique) keeping the dermal replacement submerged in the culture medium to encourage epidermal stratification and maturation (about one week).
5- Removing the artificial human skin product from the culture surface and partially dehydrating it by depositing it on a 3-5 mm thick sterile filter paper placed on a flat sterile glass surface. A fragment of porous tulle or fabric made of nylon sterilized with 70% alcohol is placed on the surface of the skin replacement to prevent the epithelial layer of the skin replacement from adhering to the filter paper. After this, a second 3-5 mm thick sterile filter paper is placed on the skin replacement covered with fabric. A flat sterile glass fragment is deposited on its surface and an object weighing approximately 250 g is placed on the latter (Figure 1). The whole process must be carried out in a laminar flow hood at room temperature for 10 minutes. The objective of this process is to significantly reduce the moisture levels of the product to reach optimum consistency and elasticity levels.

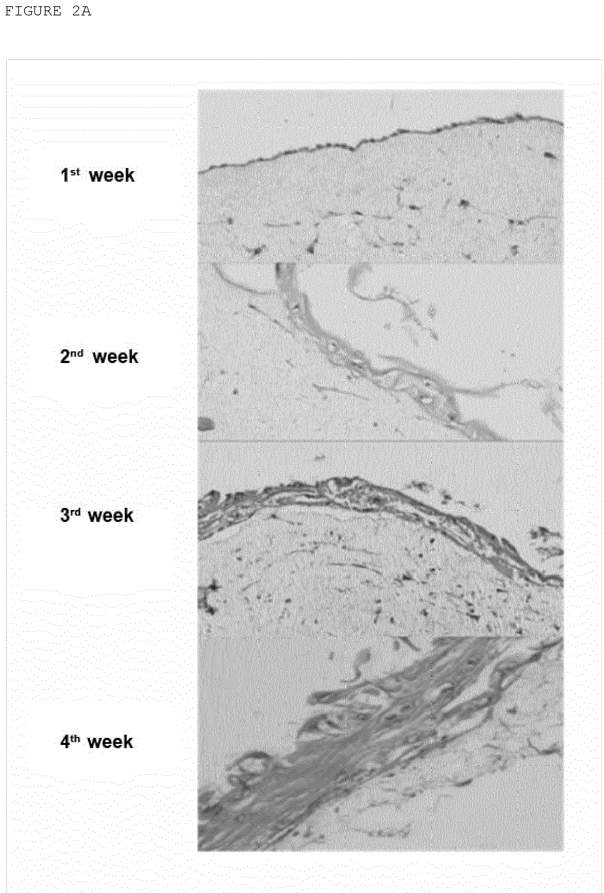

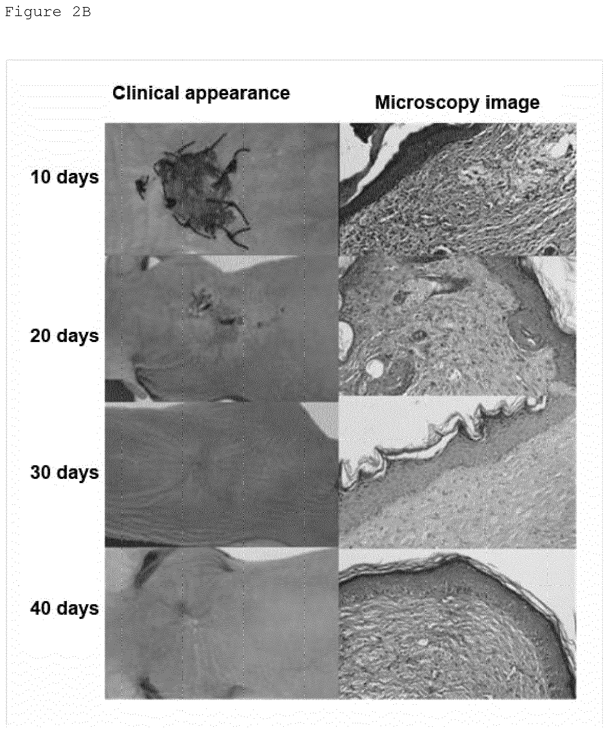

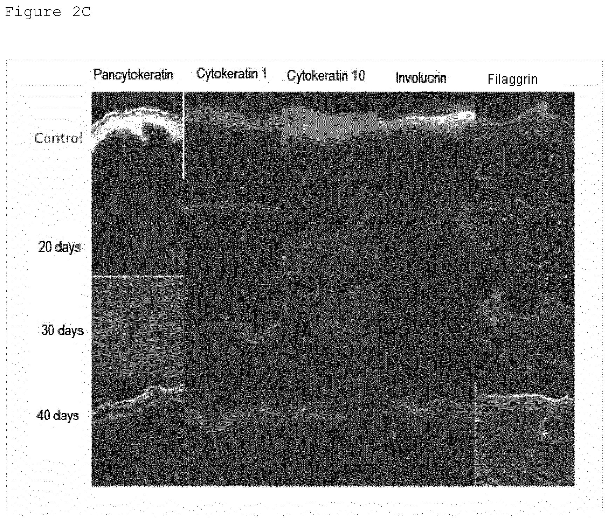

### Evaluation of the artificial human skin product (Figure 2):

### 1) Microscopic analysis of the artificial human skin product (histological quality control).

Artificial skin product evaluation revealed that their structure were very similar to the normal native human skin, although the time of development in culture directly influenced the structure of these artificial products. Specifically, the analysis of samples maintained in culture for four weeks revealed the following:

### in vitro evaluation (Figure 2A):

- The skin products evaluated once a week of their preparation had a thick stromal layer in which there is many proliferating fibroblast population. A single layer of epithelial cell appeared on the surface of this stromal replacement.
- In the second week of development in culture a greater number of cells was observed in the stroma, as well as an initial epithelium stratification, one or two rows of keratinocyte being formed on its surface.
- From this time, the keratinocyte proliferation continues, a new row of cells being observed in the epithelium in the third week.
- In the fourth week there were a total of 3 to 4 rows of cells, but it was not possible to distinguish the different epithelial strata. Papillae, cutaneous appendages or stratum corneum were not seen.

*in vivo* evaluation (Figure 2B): Unlike that which observed in the artificial skin products maintained in culture, the artificial skin implanted in an animal model had very adequate tissue structuring and differentiation levels as described below.
- On day ten after having implanted the product in the mouse the presence of a dermis very rich in cells and with disorganized extracellular fibres and material could be observed. The presence of leukocyte infiltrates in the dermis, as well as the neoformation of an important vascular tissue comprising arterioles, venules and capillaries must be highlighted. Between three and five cell layers are observed at the epithelial level, the stratum spinosum and stratum granulosum were not clearly seen, although the basal and an incipient stratum corneum were clearly seen. Similarly a homogenous dermoepidermal line is seen, although without the presence of papillae or cutaneous appendages.
- Twenty days after implanting in the athymic animals a reduction in the dermis fibroblast and leukocyte concentration, as well as a significant increase of the dermis fibril content were seen. The existence of between four and six epidermal keratinocyte layers was also observed, at this time four different strata being distinguished: basal, spinosum, granulosum and corneum, the stratum spinosum being the least obvious of the four. Similar to that at ten days of evolution, a homogenous dermoepidermal line is seen, although without the presence of papillae or cutaneous appendages.
- In the sample of day thirty a greater dermis fibril content than the previous days was observed, a large amount of epithelial strata being seen, there being between six and nine keratinocyte layers with a clear epithelial strata differentiation (basal, spinosum, granulosum and corneum). Papillae or cutaneous appendages were also not seen.
- Day forty of *in vivo* evolution, a greater fibre content was observed in the dermal stratum, the number of epithelial keratinocyte layers (between six and nine) being established. Papillae in dermoepidermal junction or cutaneous appendages were not obvious.

### 2) Immunohistochemical analysis of the artificial human skin product (Figure 2C).

The analysis of the expression of cytokeratins (pancytokeratin, cytokeratin 1 and cytokeratin 10), filaggrin and involucrin of the normal human skin controls and the skin products obtained in the laboratory, allowed determining a specific pattern of expression of these proteins for each type of sample, demonstrating that the artificial human skin is capable of expressing the same surface proteins as the normal human skin.

### Example 2. Preparing an artificial human skin product using Wharton's jelly stem cells

### A.-Obtaining skin and human umbilical cord samples.

Full thickness skin samples obtained from donors under local and locoregional anesthesia are used. Once the sample is sterilely obtained, the subcutaneous fatty tissue will be removed with the aid of scissors until exposing the dermis layer. The removed tissues will then be immediately introduced in a sterile transport medium made up of Dulbecco's Modified Eagle Medium (DMEM) supplemented with antibiotics (500 U/ml of penicillin G and 500 pg/ml of streptomycin) and antimycotic agents (1.25 pg/ml of amphotericin B) to prevent a possible sample contamination.

The umbilical cords used are obtained from the cesarean birth of normal term pregnancies. After each birth a 10-15 cm fragment of the umbilical cord is obtained which is immediately taken to the laboratory in a transport medium similar to that used for the skin.

### B.- Generating primary fibroblast and Wharton's jelly stem cell cultures.

After the transport period, all the samples must be washed two times in a sterile PBS solution with penicillin, streptomycin and amphotericin B (500 U/ml, 500 µg/ml and 1.25 µg/ml, respectively) to remove all the blood, fibrin, fat or foreign material residues which may adhere to the samples. Subsequently, each type of sample is independently processed:
- In the case of skin, the dermis is first separated from the epidermis by means of incubating the samples at 37°C in a sterile solution with 2 mg/ml dispase II in PBS. The basal membrane on which the epithelium anchors to the dermis is thus disintegrated, therefore after this, on one hand, the epithelium and on the other, the dermis will be mechanically separated.

To digest the extracellular matrix of the skin's dermis and separate the stromal fibroblasts included in said matrix, the samples must be incubated at 37°C in a 2 mg/ml a sterile solution of *Clostridium hystoliticum* type I collagenase in fetal bovine serum-free DMEM culture medium for 6 hours. This solution is capable of digesting the dermis collagen and freeing the stromal fibroblasts. To obtain primary fibroblast cultures, the digestion solution containing the digested stromal cells of the dermis must be centrifuged at 1,000 rpm for 10 minutes and the cell pellet corresponding to the fibroblasts is cultured in culture flasks having 15 cm² of surface area. Glucose enriched DMEM supplemented with antibiotics and antimycotic agents (100 U/ml of penicillin G, 100 pg/ml of streptomycin and 0.25 pg/ml of amphotericin B) and 10% fetal bovine serum (FBS) is used as the culture medium. This basic culture medium is called fibroblast medium.
- In the case of umbilical cord, the samples are sectioned longitudinally through the umbilical vein. The arteries and the umbilical vein are carefully removed in order to separate the Wharton's jelly. The jelly is subsequently fragmented until becoming very small tissue fragments. These Wharton's jelly fragments are incubated in 30 ml of type I collagenase (Gibco BRL Life Technologies, Karlsruhe, Germany) at 37°C under stirring for approximately 4-6 hours, for subsequently collecting the dissociated cells by means of centrifugation for 7 min at 1050 revolutions per minute (rpm). The supernatant is then carefully removed and the cell pellet is resuspended in 5-10 ml of prediluted trypsin (Sigma-Aldrich). It is again incubated at 37°C in a shaking bath for 30 minutes. The effects of the trypsin are then neutralized by adding 10-20 ml of culture medium supplemented with 10% fetal bovine serum, it being centrifuged again at 1000 rpm for 10 minutes to obtain the isolated cells. These cells are finally resuspended in Amniomax culture medium in a 25 cm² culture flask.

In any case the cells will be incubated at 37°C with 5% carbon dioxide in standard cell culture conditions. The culture media are renewed every three days. C.- Subculturing the cells originating from primary skin cultures and Wharton's jelly stem cell cultures .

Once the cell confluence is reached the different fibroblast cultures or Wharton's jelly stem cell cultures must be washed with sterile PBS and incubated in 1-3 ml of a solution of 0.5 g/l trypsin and 0.2 g/l EDTA at 37°C for 10 minutes.

Therefore, the cell adhesion mechanisms is disintegrated and separated cells not adhered to the surface of the culture flask are obtained.

Once the cells detached from the surface of the culture flasks, the trypsin used is inactivated by means of adding 10 ml of fibroblast culture medium. The presence of abundant serum proteins is capable of inactivating the proteolytic action of trypsin.

The solutions in which the detached cells are found, are subsequently centrifuged at 1000 rpm for 10 minutes to obtain a cell pellet or cluster with the cells of interest, the supernatant with trypsin being discarded. The cell pellet is carefully resuspended in 5 ml of culture medium and these cells are cultured in culture flasks having 15, 25 or 75 cm² of surface area.

Normally, the keratinocyte cultures are expanded until approximately five times in new culture flasks. D.- Constructing fibrin- and agarose-based artificial human skin by means of tissue engineering.
1- Generating dermis replacements with fibroblasts immersed therein using extracellular fibrin and agarose matrices. These dermal replacements will be directly generated on porous inserts of 0.4 µm in diameter to allow the passage of nutrients but not of the cells themselves. 10 ml of dermal replacement will be prepared in the following manner:
   - obtaining 7.6 ml of human blood plasma from donations (possibility of autologous origin).
   - adding 150,000 human skin fibroblasts which were previously cultured and resuspended in 750 µl of DMEM culture medium.
   - adding 150 µl of tranexamic acid to prevent the fibrinolysis of fibrin gel.
   - adding 1 ml of 1 % ClCa₂ to induce the coagulation reaction and generate a fibrin fibre network.
   - quickly adding 0.5 ml of 2% agarose type VII dissolved in PBS and heating until reaching the melting point and gently mixing by means of stirring. The final agarose concentration in the mixture will be 0.1%. The agarose concentration range allowing viable dermal products ranges between 0.025% and 0.3% and it must be established in relation to the patient object of use.
   - aliquoting as soon as possible in the porous cell culture inserts.
   - leaving it to polymerize at rest for at least 30 min.
2- Developing an epithelium layer on the surface of the dermal replacement by means of subculturing the Wharton's jelly stem cells on the dermal replacement. Covering with culture medium specific for keratinocytes. This medium is made up of 3 parts of glucose rich DMEM medium and one part of Ham F-12 medium, all of this supplemented with 10% fetal bovine serum, antibiotics-antimycotic agents (100 U/ml of penicillin G, 100 pg/ml of streptomycin and 0.25 pg/ml of amphotericin B), 24 µg/ml of adenine and different growth factors: 0.4 µg/ml of hydrocortisone, 5 µg/ml of insulin, 10 ng/ml of epidermal growth factor (EGF), 1.3 ng/ml of triiodothyronine and 8 ng/ml of cholera toxin.
3- Maintaining it in an incubator at 37°C with 5% CO2 at humid environment following standard cell culture protocols. The culture media are renewed every 3 days until the epithelial cells reach confluence on the surface of the dermal replacement (about one week).
4- Exposing the epithelial surface to air (air-liquid technique) keeping the dermal replacement submerged in the culture medium to encourage surface layer epithelial stratification and maturation (about one week).
5- Removing the artificial human skin product from the culture surface and partially dehydrating it by depositing it on a 3-5 mm thick sterile filter paper placed on a flat sterile glass surface. A fragment of porous tulle or fabric made of nylon sterilized with 70% alcohol is placed on the surface of the skin replacement to prevent the epithelial layer of the skin replacement from adhering to the filter paper. After this, a second 3-5 mm thick sterile filter paper is placed on the skin replacement covered with fabric. A flat sterile glass fragment is deposited on its surface and an object weighing approximately 250 g is placed on the latter (Figure 1). The whole process must be carried out in a laminar flow hood at room temperature for 10 minutes. The objective of this process is to significantly reduce the moisture levels of the product to reach optimum consistency and elasticity levels.

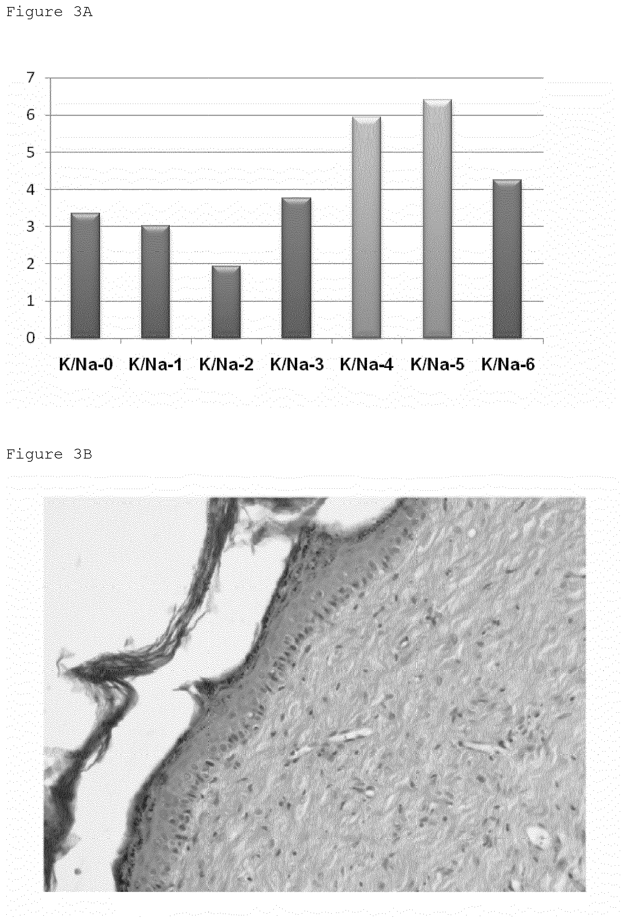

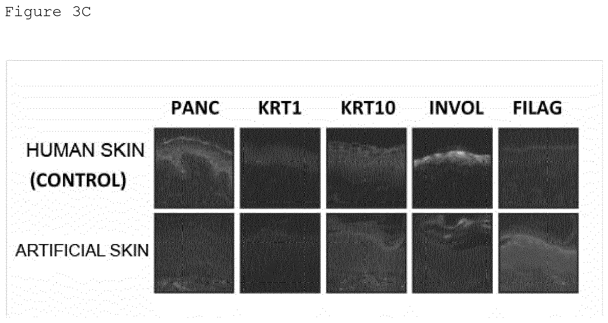

### Evaluation of the artificial human skin product with Wharton's jelly cells (Figure 3):

### 1) Determining the viability of Wharton's jelly stem cells (microanalytical quality control) (Figure 3A).

Determining cell viability by means of the energy-dispersive x-ray microanalysis techniques allows determining the intracellular Na, Mg, P, Cl, K, S and Ca ion concentration profile, and therefore knowing the subculture most suitable for its subsequent use in constructing artificial tissues. To that end it is necessary to perform the following methodology.
a) culturing the cells on gold grids previously coated with a layer of resin (pioloform) . Once the subconfluence of the cells is reached, the grids are washed with distilled water at 4°C for 10 seconds to remove the culture medium.
b) cryofixing the cells in liquid nitrogen.
c) drying the cryofixed samples by means of vacuum freeze-drying technique at low temperature (-100°C) for 20 hours. Cryofixing is performed in a Polaron E5300 freeze dryer.
d) mounting the cryofixed and dried samples in specific sample holders.
e) coating the cells with carbon in a Sputtering Polaron E-5000.
f) observing under a Philips XL-30 Scanning Electron Microscope provided with an energy-dispersive x-ray detector (EDAX), and a retrodispersed electron detector.
g) qualitative microanalytical analysis using the following constants: 10 Kv voltage, 10,000 x magnification, 0° surface angle, 35° viewing angle, 500 cps, count accumulation time: 200 seconds. The qualitative spectra are thus obtained for each studied cell. In said spectra the levels of Na, Mg, P, Cl, K, Ca in their K orbitals are selected, counts per second (CPS), the background (BKGD) or non-characteristic radiations and the peak/background (P/B) index being counted.
h) quantitative microanalytical analysis. The concentrations of the elements Na, Mg, P, Cl, S, K, and Ca in mmol/Kg of dry weight are first quantified by means of modifying the Hall method (Hall et *al.,* 1973; Staham and Pawley, 1978). To that end standard Na, Mg, P, Cl, K, S and Ca salts dissolved in 20% dextran (300,000 Dalton) are used, a standard curve or line being obtained. These salts are treated in the same manner as the specimens to be analyzed. The concentrations of each of the elements analyzed are finally calculated using the linear regression method from the standard curves.

The results obtained after quantifying the ion levels in the Wharton's jelly stem cells clearly show that the highest levels of the K/Na index correspond to the fourth and fifth subcultures and therefore, these are considered the most ideal for use in producing human skin by means of tissue engineering.

### 2) Microscopic analysis of the artificial human skin products (histological quality control) (Figure 3B).

Evaluation of the artificial human skin products with Wharton's jelly cells revealed that their structure were very similar to the normal native human skin, although the time of development in culture directly influenced the structure of these artificial tissues. Specifically, the analysis of samples maintained in culture for four weeks revealed the progressive formation of up to 5 epithelium layers on the surface of the construct, although the intercellular junctions were not formed until the last phases. The subsequent *in vivo* evaluation of the human skin products generated with Wharton's jelly stem cells showed the development of a thick epithelium layer on the artificial stroma, many desmosome-type intercellular junctions and a well-made basal membrane being formed. All of this resulted in an artificial skin product indistinguishable from normal native human skin, demonstrating the usefulness of the product generated.

### 3) Immunohistochemical analysis of the artificial human skin product (Figure 3C).

The analysis of the expression of cytokeratins (pancytokeratin, cytokeratin 1 and cytokeratin 10), filaggrin and involucrin of the normal human skin controls and the skin products obtained in the laboratory from Wharton's jelly stem cells, allow determining a specific pattern of expression of these proteins for each type of sample, demonstrating that the artificial human skin products are capable of expressing the same surface proteins as the normal human skin.

### Example 3. Preparing artificial corneas.

### Protocol for preparing artificial corneas:

The protocol described below is similar in the human and animal corneal product, with the exception of incorporating the endothelial stratum in the animal corneal product.
A. Generating primary corneal cell cultures. To establish primary corneal epithelium, stromal keratocyte and corneal endothelium cultures, where appropriate, the following methods and protocols were used:
   - Obtaining a biopsy from the sclerocorneal limbus under local or general anesthesia and transporting to the laboratory in sterile physiological saline.
   - Surgically scrubbing the cornea to remove iris, conjunctiva and blood clot residues.
   - In animal corneal product, surgically dissecting the Descemet's membrane to isolate the epithelial cells, which are cultured in medium for endothelial cells. The composition of this medium is the following: 3 parts of DMEM medium and one part Ham F-12 medium, all of this supplemented with 10% fetal bovine serum, antibiotics-antimycotic agents, 24 pg/ml of adenine and different growth factors: 0.4 pg/ml of hydrocortisone, 5 µg/ml of insulin, 1.3 ng/ml of triiodothyronine and 8 ng/ml of cholera toxin.
   - Dissecting 2 mm of central cornea and incubating in 2% collagenase I for 6 h at 37°C. Centrifuging at 1000 rpm for 10 min to collect the adult stem cells of the corneal stroma (keratocytes), which will be cultured in DMEM medium (Dulbecco's modified Eagle's medium) with 10% fetal bovine serum and antibiotics.
   - Fragmenting the sclerocorneal limbus in small explants of approximately 1 mm and culturing these explants directly on the surface of culture flasks for obtaining primary corneal epithelial cell cultures. The culture medium to be used (epithelial cell medium) is made up of 3 parts of DMEM medium and one part of Ham F-12 medium, all of this supplemented with 10% fetal bovine serum, antibiotics-antimycotic agents, 24 µg/ml of adenine and different growth factors: 0.4 µg/ml of hydrocortisone, 5 µg/ml of insulin, 1.3 ng/ml of triiodothyronine, 8 ng/ml of cholera toxin and 10 ng/ml of epidermal growth factor (EGF).

All the cultures are maintained in an incubator at 37°C with 5% CO2 at humid environment following standard cell culture protocols. The culture media are renewed every 3 days until the cells reach confluence in culture.

### B. Constructing the corneal products by means of tissue engineering in laboratory.

1 - Subculturing the endothelial cells previously isolated, using to that end porous inserts of 0.4 µm in diameter to allow the passage of nutrients but not of the cells themselves. The cells selected must belong to the fourth subculture (maximum endothelial cell viability). This first step will only be performed to generate the trilaminar animal corneal product.
2- Generating corneal stroma replacements with keratocytes immersed therein using extracellular fibrin and agarose matrices on the porous inserts of 0.4 µm in diameter (this will be the first step in the case of bilaminar human corneal product). To prepare 10 ml stroma replacement:
   - obtaining 7.6 ml of human blood plasma from donation (possibility of autologous origin).
   - adding 150,000 human keratocytes which were previously cultured and resuspended in 750 µl of DMEM culture medium.
   - adding 150 µl of tranexamic acid to prevent the fibrinolysis of fibrin gel.
   - adding 10 µl of fibronectin at a concentration of 500 mg/ml. The object is to favor the adhesion of the surface epithelial cells to the stroma replacement, eliminating the risk of detachment existing in the corneal products currently developed.
   - adding 1 ml of 1% ClCa2 to induce the coagulation reaction and generate a fibrin fibre network.
   - quickly adding 0.5 ml of 2% agarose type VII dissolved in PBS and heating until reaching the melting point and gently mixing by means of stirring. The final agarose concentration in the mixture will be 0.1%. The agarose concentration range allowing viable dermal products ranges between 0.025% and 0.3% and must be established in relation to the patient object of use.
   - aliquoting as soon as possible in the porous cell culture inserts.
   - leaving it to polymerize at rest for at least 30 min.
3- Developing a corneal epithelium layer on the surface of the corneal stroma replacement by means of subculturing the corneal epithelial cells on the stromal replacement. Covering with culture medium specific for epithelial cells.
4- Maintaining it in an incubator at 37°C with 5% CO2 at humid environment following standard cell culture protocols. The culture media are renewed every 3 days until the epithelial cells reach confluence on the surface of the stromal replacement (about one week).
5- Exposing the epithelial surface to air (air-liquid technique) maintaining the stromal replacement submerged in culture medium to encourage corneal epithelium stratification and maturation (about one week).
6- Removing the artificial human cornea product from the culture surface and partially dehydrating it by depositing it on a 3-5 mm thick sterile filter paper placed on a flat sterile glass surface. A fragment of porous tulle or fabric made of nylon sterilized with 70% alcohol is placed on the surface of the corneal replacement to prevent the epithelial layer of the corneal replacement from adhering to the filter paper. After this a second 3-5 mm thick sterile filter paper is placed on the corneal replacement covered with fabric. A flat sterile glass fragment is deposited on its surface and an object weighing approximately 250 g is placed on the latter (Figure 1). The whole process must be carried out in a laminar flow hood at room temperature for 10 minutes. The objective of this process is to significantly reduce the moisture levels of the product to reach optimum consistency and elasticity levels.

### Evaluation of the corneal products (Figure 4):

### 1) Microscopic and immunohistochemical analysis of the corneal products (histological quality control) (Figure 4A).

The microscopic analysis revealed that the corneal products generated by means of tissue engineering were structurally similar to the native corneas used as control. Specifically, a well formed corneal epithelium could be seen, with many intercellular junctions and a stroma made up of many fibrin fibres between among which include keratocytes. All of this suggests that the bilaminar or trilaminar artificial corneal products made based on the protocol described above are compatible with orthotipic human and animal corneas.

With respect to the epithelium structure of the corneal products, it should be highlighted that all the epithelial cells expressed high levels of cytokeratins typical and exclusive of the corneal epithelium (CK3 and CK1 2), which suggests that these cells could be functional *in vitro.* Similarly, the analysis of proteins related with intercellular junctions revealed the sequential expression of different desmosome components (plakoglobin, desmoglein 3 and desmoplakin), the tight junctions (ZO-1 and ZO-2) and the gap communicating junctions (connexin 37) at the epithelial level, all of this being similar to the normal native cornea.

### 2) Rheological quality control (Figure 4B).

The analysis of the mechanical properties of the bilaminar or trilaminar artificial corneal products conducted based on the protocol described above showed a viscoelastic behavior of said tissues, with an increasing elasticity modulus in the corneas with greater agarose content and a significantly lower yield point when the agarose was used at lower concentrations. The viscosimetry and yield point analyses revealed that the physical characteristics of the fibrin and agarose products were greater in those which only contained fibrin or collagen. All of this suggests that the physical properties of the fibrin and agarose corneas are optimum and are partially similar to those of the normal human corneas used as control.

### 3) Optical quality control (Figure 4C).

The optical properties of the artificial corneal products were suitable for a tissue which must perform the functions of the human or animal cornea. Specifically, the spectral transmittance analyses showed that the corneal products tended to show very suitable levels of transparency, comparables to those of the normal human cornea, with very similar scattering levels. Furthermore, the coefficients of absorption, dispersion and extinction of the fibrin and agarose products were greater than those obtained with the fibrin tissues. However, the absorbance for short wavelengths (ultraviolet range) was less in the bilaminar or trilaminar artificial corneal products than in the controls, which suggests the need of using ultraviolet light filters in products of this type. Overall, the translucency of the corneal products was acceptable, particularly in those products the thickness of which did not exceed 0.7 mm. 4)Genetic quality control (Figure 4C and 4D).

For gene expression analysis the total RNA was extracted from the primary corneal cultures of human epithelial and stromal keratocyte cells and of the human bilaminar artificial corneal products, the latter being analyzed by means of microarray (Affymetrix Human Genome U133 plus 2.0®). This analysis demonstrated that the genes which were expressed in the human artificial corneal products were compatibles with normal corneal function, although many genes related to tissue development were overexpressed in relation to the normal cornea. Specifically, the artificial corneal products expressed a large number of genes the function of which was centered in establishing intercellular junctions (connexins, integrins, desmoplakin, plakoglobin, etc.), epithelial development (Sema3A, RUNX2, TBX1), cell differentiation (PLXNA4A, FLG, DKK4, DCN), basal membrane (laminins, collagen IV), extracellular matrix (collagens, decorin, biglycan, MMP, fibronectin), etc. These results suggest that the corneal products made could be undergoing a development process similar to that occurring in the normal cornea and that the gene functions expressed by these products are compatibles with the norm.

### 4) in vivo evaluation (Figure 4E).

To evaluate the clinical behavior of the artificial corneal products 6 bilaminar human corneas of partial thickness were implanted in laboratory rabbits and their evolution was tracked for 6 months. The results of this assay show suitable biocompatibility levels, with a complete absence of inflammation or infection, maintaining good levels of transparency. All of this suggests that the corneal products generated by means of tissue engineering could potentially be useful from a experimental pharmacological and clinical view point.

### Example 4. Preparing an artificial human urethra product.

### Protocol for preparing an artificial human urethra product:

### A.-Obtaining human urethra samples.

To generate artificial urethras, small biopsies of normal human urethra obtained by means of endoscopy of normal patients or donors are used. Once the sample is sterilely obtained, the removed tissues will be immediately introduced in a sterile transport medium made up of Dulbecco's Modified Eagle Medium (DMEM) supplemented with antibiotics (500 U/ml of penicillin G and 500 pg/ml of streptomycin) and antimycotic agents (1.25 pg/ml of amphotericin B) to prevent a possible sample contamination.

Alternatively, in the cases in which taking human urethra samples is not feasible, oral mucosa samples or skin samples can be used to generate urethra replacements.

### B.- Generating primary stromal and epithelial cell cultures.

After the transport period all the samples must be washed two times in a sterile PBS solution with penicillin, streptomycin and amphotericin B (500 U/ml, 500 µg/ml and 1.25 µg/ml, respectively) to remove all the blood, fibrin, fat or foreign material residues which may adhere to the samples.

First, to separate the stroma from the epithelium the samples are incubated at 37°C in a sterile solution with trypsin 0.5 g/l and EDTA 0.2 g/l in PBS, the supernatant with the cells being collected after 30 minutes by means of centrifugation. This process is repeated up to 5 times adding new trypsin-EDTA solution each time. A significant amount of urethral epithelial cells is thus obtained, which are cultured in epithelial cell culture medium that preferably favors epithelial cell growth on fibroblasts. This medium is made up of 3 parts of Glucose rich DMEM medium and one part of Ham F-12 medium, all of this supplemented with 10% fetal bovine serum, antibiotics-antimycotic agents (100 U/ml of penicillin G, 100 µg/ml of streptomycin and 0.25 µg/ml of amphotericin B), 24 µg/ml of adenine and different growth factors: 0.4 pg/ml of hydrocortisone, 5 pg/ml of insulin, 10 ng/ml of epidermal growth factor (EGF), 1.3 ng/ml of triiodothyronine and 8 ng/ml of cholera toxin.

To digest the extracellular matrix of the urethral stroma and separate the stromal fibroblasts included in said matrix, the samples must be incubated at 37°C in a 2 mg/ml sterile solution of *Clostridium hystoliticum* type I collagenase in fetal bovine serum-free DMEM culture medium for 6 hours. This solution is capable of digesting the dermis collagen and freeing the stromal fibroblasts. To obtain primary fibroblast cultures, the digestion solution containing the digested stromal cells of the dermis must be centrifuged at 1,000 rpm for 10 minutes and the cell pellet corresponding to the fibroblasts is cultured in culture flasks having 15 cm² of surface area. Glucose enriched DMEM supplemented with antibiotics and antimycotic agents (100 U/ml of penicillin G, 100 pg/ml of streptomycin and 0.25 pg/ml of amphotericin B) and 10% fetal bovine serum (FBS) is used as the culture medium. This basic culture medium is called fibroblast medium.

In all the cases the cells will be incubated at 37°C with 5% carbon dioxide, in standard cell culture conditions. The culture media are renewed every three days.

### C.- Constructing fibrin- and agarose-based artificial human urethra products by means of tissue engineering.

1- Generating stromal replacements with fibroblasts immersed therein using extracellular fibrin and agarose matrices. These stromal replacements will be directly generated on cell culture Petri dishes. 10 ml of stromal replacement will be prepared in the following manner:
   - obtaining 7.6 ml of human blood plasma from donation (possibility of autologous origin).
   - adding 150,000 human skin fibroblasts which were previously cultured and resuspended in 750 µl of DMEM culture medium.
   - adding 150 µl of tranexamic acid to prevent the fibrinolysis of fibrin gel.
   - adding 1 ml of 1% ClCa2 to induce the coagulation reaction and generate a fibrin fibre network.
   - quickly adding 0.5 ml of 2% agarose type VII dissolved in PBS and heating until reaching the melting point and gently mixing by means of stirring. The final agarose concentration in the mixture will be 0.1%. The agarose concentration range allowing viable dermal products ranges between 0.025% and 0.3% and it must be established in relation to the patient object of use.
   - aliquoting as soon as possible in the cell culture Petri dishes.
   - leaving it to polymerize at rest for at least 30 min.
2- Developing a corneal epithelium layer (epidermis) on the surface of the dermal replacement by means of subculturing the epithelial cells on the stromal replacement. Covering with culture medium specific for epithelial cells.
3- Maintaining it in an incubator at 37°C with 5% CO2 at humid environment following standard cell culture protocols. The culture media are renewed every 3 days until the epithelial cells reach confluence on the surface of the dermal replacement (about one week).
4- Removing the artificial urethra human product from the culture surface and partially dehydrating it by depositing it on a 3-5 mm thick sterile filter paper placed on a flat sterile glass surface. A fragment of porous tulle or fabric of nylon sterilized with 70% alcohol is placed on the surface of the stromal replacement to prevent the epithelial layer of the urethral replacement from adhering to the filter paper. After this a second 3-5 mm thick sterile filter paper is placed on the stromal replacement covered with fabric. A flat sterile glass fragment is deposited on its surface and an object weighing approximately 250 g is placed on the latter (Figure 1). The whole process must be carried out in a laminar flow hood at room temperature for 10 minutes. The objective of this process is to significantly reduce the moisture levels of the product to reach optimum consistency and elasticity levels.
5- Once the tissue is dehydrated, the urethra replacement is cut to measure and rolled up, it is then sutured with monofilament surgical thread. A tubular-shaped urethra replacement very similar to the native human urethra is thus obtained. It should be highlighted that dehydrating the tissue results in suitable consistency and elasticity levels, allowing rolling up and suturing without any difficulty.

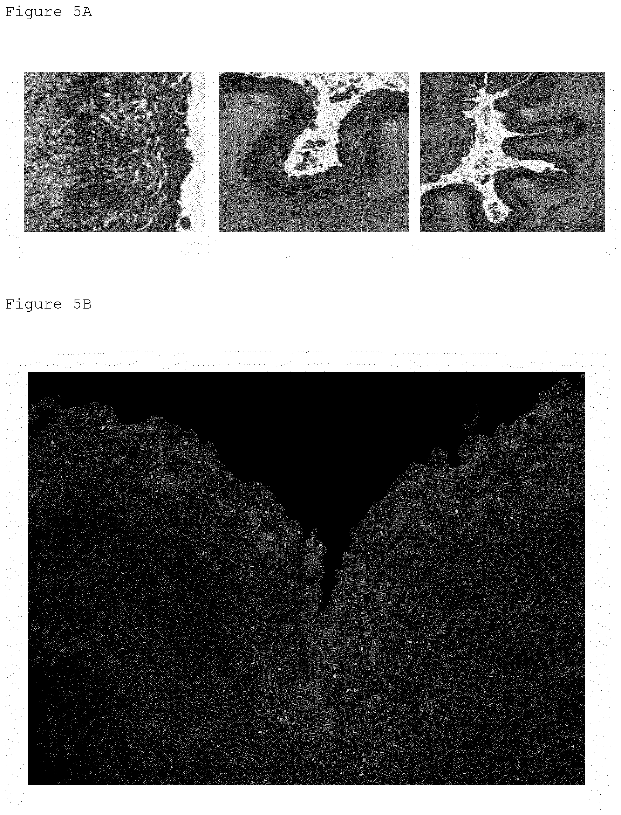

### Evaluation of the artificial human urethra product (Figure 5) :

### 1) Microscopic analysis of the artificial human urethra product (histological quality control) (Figure 5A).

Artificial urethra product evaluation revealed that their structure were very similar to the normal native human urethra. Specifically, the analysis of samples maintained in culture for four weeks revealed the formation of a stratified squamous epithelium on the surface of the artificial stroma and the generation of a dense fibre rich stroma, in which the stromal cells actively proliferated. All of this suggests that the structure of the artificial urethra was the same as that of the normal native urethra.

In those cases in which cells of the oral mucosa are used, stromal replacements very similar to those obtained from urethral cells were obtained.

### 2) Immunohistochemical analysis of the artificial human urethra product (Figure 5B).

The analysis of cytokeratin expression of the normal urethra controls and the artificial urethra products obtained in the laboratory demonstrated the expression of integrins by the artificial urethral epithelium, which suggests that this epithelium is fully functional and could be used for replacing human urethra.

### Example 5. Preparing an artificial urine bladder product

### Protocol for producing an artificial human urinary bladder product:

### A.-Obtaining human urinary bladder samples.

To generate artificial urinary bladder replacements, small biopsies of the normal human bladder obtained by means of endoscopy of normal patients or donors are used. Once the sample is sterilely obtained, the tissues removed will be immediately introduced in a sterile transport medium made up of Dulbecco's Modified Eagle Medium (DMEM) supplemented with antibiotics (500 U/ml of penicillin G and 500 pg/ml of streptomycin) and antimycotic agents (1.25 pg/ml of amphotericin B) to prevent a possible sample contamination.

Alternatively, in the cases in which taking human bladder samples is not feasible, oral mucosa samples or skin samples can be used to generate bladder replacements.

### B.- Generating primary stromal and epithelial cell cultures.

After the transport period, all the samples must be washed two times in a sterile PBS solution with penicillin, streptomycin and amphotericin B (500 U/ml, 500 µg/ml and 1.25 µg/ml, respectively) to remove all the blood, fibrin, fat or foreign material residues which could may adhere to the samples.

First, to separate the stroma from the epithelium the samples are incubated at 37°C in a sterile solution with 0.5 g/l trypsin and 0.2 g/l EDTA in PBS, the supernatant with the cells being collected after 30 minutes by means of centrifugation. This process is repeated up to 5 times adding new trypsin-EDTA solution each time. A significant amount of bladder epithelial cells is thus obtained, which are cultured in epithelial cell culture medium that preferably favors epithelial cells growth on fibroblasts. This medium is made up of 3 parts of glucose rich DMEM medium and one part of Ham F-12 medium, all of this supplemented with 10% fetal bovine serum, antibiotics-antimycotic agents (100 U/ml of penicillin G, 100 pg/ml of streptomycin and 0.25 µg/ml of amphotericin B), 24 µg/ml of adenine and different growth factors: 0.4 µg/ml of hydrocortisone, 5 µg/ml of insulin, 10 ng/ml of epidermal growth factor (EGF), 1.3 ng/ml of triiodothyronine and 8 ng/ml of cholera toxin.

To digest the extracellular matrix of the bladder stroma and separate the stromal fibroblasts included in said matrix, the samples must be incubated at 37°C in a 2 mg/ml sterile solution of *Clostridium hystoliticum* type I collagenase in fetal bovine serum-free DMEM culture medium for 6 hours. This solution is capable of digesting the stromal collagen and freeing the fibroblasts immersed therein. To obtain primary fibroblast cultures the digestion solution containing the digested stromal cells must be centrifuged at 1,000 rpm for 10 minutes and the cell pellet corresponding to the fibroblasts is cultured in culture flasks having 15 cm² of surface area. Glucose enriched DMEM supplemented with antibiotics and antimycotic agents (100 U/ml of penicillin G, 100 µg/ml of streptomycin and 0.25 µg/ml of amphotericin B) and 10% fetal bovine serum (FBS) is used as the culture medium. This basic culture medium is called fibroblast medium.

In all the cases the cells will be incubated at 37°C with 5% carbon dioxide, in standard cell culture conditions. The culture media are renewed every three days.

### C.- Constructing fibrin- and agarose-based artificial human urinary bladder products by means of tissue engineering.

1- Generating stromal replacements with fibroblasts immersed therein using extracellular fibrin and agarose matrices. These stromal replacements will be generated directly on cell culture Petri dishes. 10 ml of stromal replacement will be prepared in the following manner:
   - obtaining 7.6 ml of human blood plasma from donation (possibility of autologous origin).
   - adding 150,000 human bladder fibroblasts previously cultured and resuspended in 750 µl of DMEM culture medium.
   - adding 150 µl of tranexamic acid to prevent the fibrinolysis of fibrin gel.
   - adding 1 ml of 1% ClCa2 to induce the coagulation reaction and generate a fibrin fibre network.
   - quickly adding 0.5 ml of 2% agarose type VII dissolved in PBS and heating until reaching the melting point and gently mixing by means of stirring. The final agarose concentration in the mixture will be 0.1%. The agarose concentration range allowing viable dermal products ranges between 0.025% and 0.3% and it must be established in relation to the patient object of use.
   - aliquoting as soon as possible in the cell culture Petri dishes.
   - leaving it to polymerize at rest for at least 30 min.
2- Developing an epithelium layer (urothelium) on the surface of the stromal replacement by means of subculturing the epithelial cells on this replacement. Covering with culture medium specific for epithelial cells.
3- Maintaining it in an incubator at 37°C with 5% CO2 at humid environment following standard cell culture protocols. The culture media are renewed every 3 days until the epithelial cells reach confluence on the surface of the dermal replacement (about one week)).
4- Removing the artificial bladder human product from the culture surface and partially dehydrating it by depositing it on a 3-5 mm thick sterile filter paper placed on a flat sterile glass surface. A fragment of porous tulle or fabric of nylon sterilized with 70% alcohol is placed on the surface of the stromal replacement to prevent the epithelial layer of the bladder replacement from adhering to the filter paper. After this a second 3-5 mm thick sterile filter paper is placed on the stromal replacement covered with fabric. A flat sterile glass fragment is deposited on its surface and an object weighing approximately 250 g is placed on the latter (Figure 1). The whole process must be carried out in a laminar flow hood at room temperature for 10 minutes. The objective of this process is to significantly reduce the moisture levels of the product to reach optimum consistency and elasticity levels.
5- Once the tissue is dehydrated, the bladder replacement is cut to measure and sutured on itself giving it the desired form using monofilament surgical thread. It should be highlighted that dehydrating the tissue results in suitable consistency and elasticity levels, allowing suturing without any difficulty.

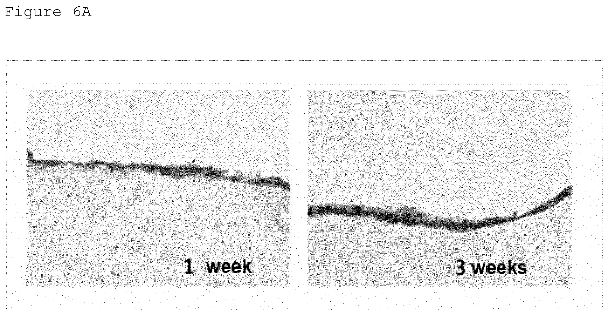

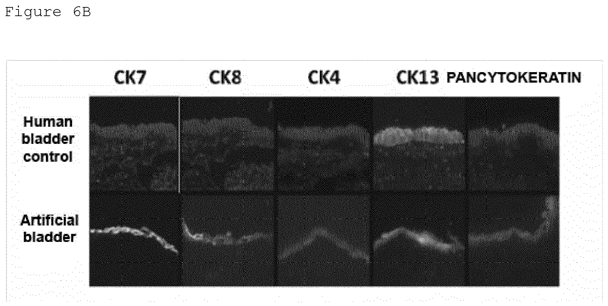

### Evaluation of the artificial human urinary bladder product (Figure 6):

### 1) Microscopic and immunohistochemical analysis of the artificial human bladder product (histological quality control) (Figures 6A and 6B).

Artificial bladder product evaluation revealed that their structure were very similar to the normal native human bladder. Specifically, the analysis of samples maintained in culture for four weeks revealed the formation of a cuboidal or squamous simple epithelium on the surface of the artificial stroma and the generating a dense fibre rich stroma, in which the stromal cells actively proliferated (Figure 6A).

The immunohistochemical analysis revealed that these tissues expressed cytokeratin 13 and pancytokeratin, similar to the normal control human bladder tissues, as well as cytokeratins 7 and 8, typical of embryonic tissues or maturing tissues (Figure 6B). All of this suggests that the structure of the artificial bladder was the same as that of the normal native bladder.

In those cases in which cells of the oral mucosa were used, stromal replacements very similar to those obtained from bladder cells were obtained.

### Example 6. Preparing artificial products by means of fibrin, agarose and collagen biomaterials.

### Protocol for preparing artificial human oral mucosa products:

### A. Generating primary epithelial and stromal cell cultures.

To establish primary cultures of epithelial cells (oral mucosal epithelial cells) and stromal cells (fibroblasts), biopsies of normal oral mucosa originating from human donors or laboratory animals are used, using the following methods and protocols:
- obtaining a biopsy of the oral mucosa and transporting to the laboratory in sterile physiological saline.
- isolating and culturing epithelial and stromal cells using standard enzymatic digestions techniques (trypsin or dispase for epithelial isolation and collagenase for stroma isolation) or standard tissue explant techniques.
- Culturing in media specific for epithelial or stromal cells until reaching cell confluence.

All the cultures are maintained in an incubator at 37°C with 5% CO2 at humid environment following standard cell culture protocols. The culture media are renewed every 3 days until the cells reach confluence in culture.

### B. Constructing fibrin, agarose and collagen tissue replacements by means of tissue engineering in laboratory.

1- Generating tissue stroma replacements with stromal cells immersed therein. To prepare 20 ml of stromal replacement:
   a.-Preparing 10 ml of liquid type I collagen by taking 8.81 ml volume of 6.4 mg/ml liquid collagen concentrate. Adding 1.1 ml of 10X PBS and adjusting the pH to 7.4±0.2 by means of adding approximately 90 µL of NaOH. To increase the pH NaOH at a concentration between 0.1 and 1 M is usually used, although other products can also be used. Mixing by means of gentle stirring and maintaining it in ice to prevent premature gelling.
   b.- Preparing 10 ml of a fibrin and agarose mixture in the following manner:
      - obtaining 7.6 ml of human blood plasma from donation (possibility of autologous origin).
      - adding 150,000 stromal cells which were previously cultured and resuspended in 750 µl of DMEM culture medium.
      - adding 150 µl of tranexamic acid to prevent the fibrinolysis of fibrin gel.
      - adding 1 ml of 1% ClCa2 to induce the coagulation reaction and generate a fibrin fibre network.
      - quickly adding 0.5 ml of 2% agarose type VII dissolved in PBS and heating until reaching the melting point and gently mixing by means of stirring. The final agarose concentration in the mixture will be 0.1%. The agarose concentration range allowing viable corneal products ranges between 0.025% and 0.3% and it must be established in relation to the patient object of use.
   c.- Mixing both solutions (collagen solution and fibrin-agarose solution) in variable proportion depending on the product to be generated. In all the cases, mixing both solutions as quickly as possible, keeping the collagen solution cold until the time of mixing.
      - adding 10 ml of the collagen solution and 10 ml of the fibrin-agarose solution to generate product A (2.8 g/L of collagen, 1.25 g/L of fibrin and 0.5 g/L of agarose).
      - adding 15 ml of the collagen solution and 5 ml of the fibrin-agarose solution to generate product B (3.8 g/L of collagen, 0.6 g/L of fibrin and 0.25 g/L of agarose).
      - adding 5 ml of the collagen solution and 15 ml of the fibrin-agarose solution to generate product C (1.9 g/L of collagen, 1.9 g/L of fibrin and 0.75 g/L of agarose).
   d.- aliquoting as soon as possible in sterile porous cell culture inserts or in Petri dishes.
   e.- leaving it to polymerize at rest for at least 30 min in an incubator at 37°C.
2- Developing an epithelium layer on the surface of the stromal replacement by means of subculturing the oral mucosal epithelial cells on the stromal replacement. Covering with culture medium specific for epithelial cells.
3- Maintaining it in an incubator at 37°C with 5% CO2 at humid environment following standard cell culture protocols. The culture media are renewed every 3 days until the epithelial cells reach confluence on the surface of the stromal replacement (about one week).
4- Exposing the epithelial surface to air (air-liquid technique) maintaining the stromal replacement submerged in culture medium to encourage epithelium stratification and maturation (about one week).
5- Removing the artificial tissue from the culture surface and partially dehydrating it by depositing it on a 3-5 mm thick sterile filter paper placed on a flat sterile glass surface. A fragment of porous tulle or fabric made of nylon sterilized with 70% alcohol is placed on the surface of the stromal replacement to prevent the epithelial layer of the tissue replacement from adhering to the filter paper. After this, a second 3-5 mm thick sterile filter paper is placed on the stromal replacement covered with fabric. A flat sterile glass fragment is deposited on its surface and an object weighing approximately 250 g is placed on the latter (Figure 1). The whole process must be carried out in a laminar flow hood at room temperature for 10 minutes. The objective of this process is to significantly reduce the moisture levels of the product to reach optimum consistency and elasticity levels.
6- Once the tissue is dehydrated, it is cut to measure giving it the desired form by using monofilament surgical thread. It should be highlighted that dehydrating the tissue results in suitable consistency and elasticity levels, allowing suturing without any difficulty.

### C. Constructing collagen tissue replacements by means of tissue engineering in laboratory.

1- Generating tissue stroma replacements with stromal cells immersed therein. To prepare 20 ml of stromal replacement:
   - Taking 17.62 ml of 6.4 mg/ml liquid collagen concentrate; adding 1.45 ml of 10X PBS and adjusting the pH to 7.4±0.2 by means of adding approximately 180 µL of NaOH. To increase the pH NaOH at a concentration between 0.1 and 1 M is usually used, although other products can also be used. Mixing by means of gentle stirring and keeping it in ice to prevent premature gelling.
   - adding 150,000 stromal cells which were previously cultured and resuspended in 750 µl of DMEM culture medium.
   - aliquoting as soon as possible in sterile porous cell culture inserts or Petri dishes.
   - leaving it to polymerize at rest for at least 30 min in an incubator at 37°C.
2- Developing an epithelium layer on the surface of the stromal replacement by means of subculturing the oral mucosal epithelial cells on the stromal replacement. Covering with culture medium specific for epithelial cells.
3- Maintaining it in an incubator at 37°C with 5% CO2 at humid environment following standard cell culture protocols. The culture media are renewed every 3 days until the epithelial cells reach confluence on the surface of the stromal replacement (about one week).
4- Exposing the epithelial surface to air (air-liquid technique) maintaining the stromal replacement submerged in culture medium to encourage epithelium stratification and maturation (about one week)..
5- Removing the artificial tissue from the culture surface and partially dehydrating it by depositing it on a 3-5 mm thick sterile filter paper placed on a flat sterile glass surface. A fragment of porous tulle or fabric made of nylon sterilized with 70% alcohol is placed on the surface of the stromal replacement to prevent the epithelial layer of the tissue replacement from adhering to the filter paper. After this, a second 3-5 mm thick sterile filter paper is placed on the stromal replacement covered with fabric. A flat sterile glass fragment is deposited on its surface and an object weighing approximately 250 g is placed on the latter (Figure 1). The whole process must be carried out in a laminar flow hood at room temperature for 10 minutes.
6- Once the tissue is dehydrated, it is cut to measure giving it the desired form by using monofilament surgical thread.

### Evaluation of the artificial human oral mucosa products (Figure 7):

The products obtained according to the protocol above were evaluated. The fibrin, agarose and collagen concentrations for each of the products prepared are indicated below:
A.- fibrin (1.25 g/L), agarose (0.5 g/L) and collagen (2.8 g/L).
B.- fibrin (0.6 g/L), agarose (0.25 g/L) and collagen (3.8 g/L).
C.- fibrin (1.9 g/L), agarose (0.75 g/L) and collagen (1.9 g/L).
D.- fibrin (0 g/L), agarose (0 g/L) and collagen (5.6 g/L) .

### 1) Microscopic analysis of the artificial tissues (histological quality control) (Figure 7A).

The microscopic analysis revealed that the artificial tissues generated by means of tissue engineering were structurally similar to the native tissues used as control. Specifically, a stroma made up of many fibres between which the stromal cells were found, showing suitable cell proliferation levels could be seen. In comparison with other models previously described (particularly, fibrin models and fibrin with agarose models), the fibrin, agarose and collagen tissues have greater fibril density at the stroma level. All of this suggests that the tissue products constructed based on the protocol described above are compatible with the normal native human tissues. 2) Rheological quality control (Figure 7B).

Analyzing the biomechanical properties of the tissue products constructed based on the protocol described above by means of a rheometer. The results are shown in Figure 7C and they reveal that the best tissue is product A followed by C, B being very similar to D (only collagen). Therefore the analysis demonstrated an improvement of viscoelastic behavior and an increasing threshold stress as the collagen concentration increases higher than that shown by the artificial collagen tissues.

The data presented in these examples show that the physical properties of the fibrin, agarose and collagen tissues are optimum and similar to those of the normal human tissues used as control, which entails an improvement with respect to the artificial tissues described with respect to their clinical use.

### Example 8: Improving the fibrin-agarose biomaterials after applying structuring techniques.

The application of nanostructuring techniques is capable of substantially modifying the structure and behavior of the biomaterials subjected to this process. The main effects of nanostructuring on 0.1% fibrin and agarose biomaterial (final preferred concentration of the patent application) are the following:
- Significant improvement of the biomechanical properties of the tissue. This increase allows manipulating the nanostructured tissue and entails a substantial and unexpected improvement of the rheological properties of the biomaterial. As shown in Figure 8, the threshold stress was 3.8 times greater in the nanostructured tissues with respect to the non-nanostructured tissues (16.2 and 4.23 Pascals, respectively). Meanwhile, the viscous modulus G" of the samples subjected to nanostructuring was 5.26 times greater with respect to the non-nanostructured samples (19.3 and 3.67 Pa, respectively) (Figure 9). These data indicate that the resistance of the nanostructured biomaterials is much greater than that of the non-nanostructured tissues.

Similarly, the elastic modulus G' of the nanostructured tissues was 5.55 times greater (Figure 10) with respect to the native non-nanostructured biomaterials (152 and 27.4 Pa, respectively), which means that these tissues have significantly higher elasticity levels.

All these phenomena are very significant and clearly do not depend on the water concentration in the biomaterial, but on the internal reactions generated in the biomaterial as a result of the nanostructuring process.
- Substantial improvement of the manipulability of the nanostructured tissue which allowed its surgical manipulation, suturing to the recipient bed and implanting in test animals. It is important to highlight that the non-nanostructured tissues were significantly more difficult to manipulate, tending to break and considerably complicating clinical suturing and implanting. This improvement is very significant and it cannot be simply explained by the reduction of water concentration in the biomaterial, but by the formation of three-dimensional structures binding the different fibrin and agarose fibres, the properties of the biomaterial being unexpectedly modified.
- Significant improvement of the transparency of the tissues subjected to nanostructuring. The transparency of the 0.1% non-nanostructured fibrin-agarose biomaterials, measured as the transmittance percentage of the visible light spectrum (from approximately 400 to 700 nm), reached levels of 90-94% depending on the wavelength considered (mean 92%), whereas the non-nanostructured biomaterials had a transmittance of 87-90% (mean 88.5%) (Figure 10). Therefore there is a better transparency (greater transmittance of visible light) in the biomaterials subjected to the nanostructuring process. Similar to the previous cases, this phenomenon is the result of a complex process of chemical and physical reactions in the internal structure of the biomaterial and an improvement in transparency is not completely predictable given that the nanostructured tissues are denser and have a lower water content than the non-nanostructured tissues.
- Clinical result once implanted in laboratory animals. Implanting the biomaterials subjected to nanostructuring has shown to be more effective from a clinical view point than that of the non-structured biomaterials. This fact, which is not predictable in advance, must be related to, on one hand, the greater clinical implant efficiency due to the suitable biomechanical properties of the biomaterial and, on the other hand, to the fact that the biomaterials subjected to nanostructuring have a greater fibre density per mm² and, therefore, a slower remodelization by the receiving organism.

## Claims

1. An *in vitro* method for preparing an artificial tissue comprising:
a)adding a composition comprising fibrinogen to a sample of isolated cells,
b)adding an antifibrinolytic agent to the product resulting from step (a),
c)adding at least one coagulation factor, a source of calcium, thrombin, or any combination of the above to the product resulting from step (b),
d)adding a composition of a polysaccharide to the product resulting from step (c),
e)culturing isolated cells in or on the product resulting from step (d), and
f)inducing the nanostructuring of the product resulting from step (e), wherein said nanostructuring induction comprises the dehydration and/or mechanical compression of the product resulting from step (e), wherein the polysaccharide of step (d) is agarose.

2. The method according to claim 1, wherein the cells of step (a) are fibroblasts or keratocytes.

3. The method according to any of claims 1 to 2, further comprising a step (step b2) between steps (b) and (c) wherein a protein is added.

4. The method according to claim 3, wherein the protein added in step (b2) is fibronectin.

5. The method according to any of claims 1 to 4, comprising a step (d2) between steps (d) and (e) which comprises adding a composition comprising a protein to the product resulting from step (d).

6. The method according to claim 5, wherein the protein added in step (d2) is collagen.

7. The method according to any of claims 1 to 6, wherein the cells of step (e) comprise umbilical cord stem cells.

8. The method according to any of claims 1 to 7, wherein the cells of step (e) comprise epithelial cells.

9. The method according to claim 1, wherein the dehydration of the product resulting from step (e) comprises a method selected from the list comprising: draining, evaporation, suction, capillary pressure, osmosis or electro-osmosis.

10. The method according to claim 9, wherein the dehydration of the product resulting from step (e) by means of capillary pressure comprises the application of an absorbent material on the product resulting from step (e).

11. The method according to any of claims 1 to 10, wherein the mechanical compression of step (f) comprises a method selected from the list comprising: application of a static load, application of a hydraulic, application of a cam, application of one or more rollers, application of a balloon, extrusion or centrifugation.

12. The method according to claim 11, wherein the application of a static load of step (f) comprises placing a weight on the product resulting from step (e).

13. The method according to any of claims 1 to 12, wherein between step (e) and step (f) there is an additional step in which the product resulting from step (e) is exposed to air.

14. An artificial tissue obtainable by the method according to any of claims 1 to 13.

15. Use of the artificial tissue according to claim 14, for evaluating a pharmacological and/or chemical product.

16. The artificial tissue according to claim 14 for use in medicine.

17. The artificial tissue according to claim 14, for use in partially or completely increase, restore or replace the functional activity of a diseased or damaged tissue or organ.

18. The artificial tissue for use according to claim 17, wherein the damaged tissue or the organ is selected from the list comprising: skin, bladder, urethra, cornea, mucosa, conjunctiva, abdominal wall, conjunctiva, eardrum, pharynx, larynx, intestine, peritoneum, ligament, tendon, bone, meninx or vagina.

19. A pharmaceutical composition comprising the artificial tissue according to claim 14.

## Patentansprüche

1. *In vitro*-Verfahren zur Herstellung eines künstlichen Gewebes umfassend:
a) Hinzufügen einer Zusammensetzung, die Fibrinogen umfasst, zu einer Probe isolierter Zellen,
b) Hinzufügen eines antifibrinolytischen Agens zu dem Produkt, das sich aus Schritt (a) ergibt,
c) Hinzufügen von mindestens einem Koagulationsfaktor, einer Calciumquelle, Thrombin, oder einer beliebigen Kombination hiervon zu dem Produkt, das sich aus Schritt (b) ergibt,
d) Hinzufügen einer Zusammensetzung eines Polysaccharids zu dem Produkt, das sich aus Schritt (c) ergibt,
e) Züchten isolierter Zellen in oder auf dem Produkt, das sich aus Schritt (d) ergibt, und
f) Herbeiführen der Nanostrukturierung des Produkts aus Schritt (e), wobei das Herbeiführen der Nanostrukturierung das Dehydrieren und/oder das mechanische Komprimieren des Produkts, das sich aus Schritt (e) ergibt, umfasst, wobei das Polysaccharid von Schritt (d) Agarose ist.

2. Verfahren nach Anspruch 1, wobei die Zellen von Schritt (a) Fibroblasten oder Keratozyten sind.

3. Verfahren nach einem der Ansprüche 1 bis 2, das des Weiteren einen Schritt (Schritt b2) zwischen den Schritten (b) und (c) umfasst, wobei ein Protein hinzugefügt wird.

4. Verfahren nach Anspruch 3, wobei das in Schritt (b2) hinzugefügte Protein Fibronektin ist.

5. Verfahren nach einem der Ansprüche 1 bis 4, das einen Schritt (d2) zwischen den Schritten (d) und (e) umfasst, der das Hinzufügen einer Zusammensetzung, die ein Protein umfasst, zu dem Produkt, das sich aus Schritt (d) ergibt, umfasst.

6. Verfahren nach Anspruch 5, wobei das in Schritt (d2) hinzugefügte Protein Kollagen ist.

7. Verfahren nach einem der Ansprüche 1 bis 6, wobei die Zellen von Schritt (e) Nabelschnurstammzellen umfassen.

8. Verfahren nach einem der Ansprüche 1 bis 7, wobei die Zellen von Schritt (e) Epithelzellen umfassen.

9. Verfahren nach Anspruch 1, wobei das Dehydrieren des Produkts, das sich aus Schritt (e) ergibt, ein Verfahren umfasst, ausgewählt aus der Liste umfassend: Entwässern, Verdampfen, Absaugen, Kapillardruck, Osmose oder Elektroosmose.

10. Verfahren nach Anspruch 9, wobei das Dehydrieren des Produkts, das sich aus Schritt (e) ergibt, durch Kapillardruck das Aufbringen eines Absorptionsmaterials auf das Produkt, das sich aus Schritt (e) ergibt, umfasst.

11. Verfahren nach einem der Ansprüche 1 bis 10, wobei das mechanische Komprimieren von Schritt (f) ein Verfahren umfasst, ausgewählt aus der Liste umfassend: Aufbringen einer statischen Last, Anwenden einer Hydraulik, Anwenden einer Nocke, Anwenden einer oder mehrerer Walzen, Anwenden eines Ballons, Extrusion oder Zentrifugation.

12. Verfahren nach Anspruch 11, wobei das Aufbringen einer statischen Last von Schritt (f) das Anordnen eines Gewichts auf dem Produkt, das sich aus Schritt (e) ergibt, umfasst.

13. Verfahren nach einem der Ansprüche 1 bis 12, wobei zwischen Schritt (e) und Schritt (f) ein zusätzlicher Schritt ist, in dem das Produkt, das sich aus Schritt (e) ergibt, Luft ausgesetzt wird.

14. Künstliches Gewebe, das durch das Verfahren nach einem der Ansprüche 1 bis 13 erhältlich ist.

15. Verwendung des künstlichen Gewebes nach Anspruch 14 zur Bewertung eines pharmakologischen und/oder chemischen Produkts.

16. Künstliches Gewebe nach Anspruch 14 zur Verwendung in der Medizin.

17. Künstliches Gewebe nach Anspruch 14 zur Verwendung bei teilweiser oder vollständiger Erhöhung, Wiederherstellung oder Ersetzung der funktionellen Aktivität eines erkrankten oder geschädigten Gewebes oder Organs.

18. Künstliches Gewebe zur Verwendung nach Anspruch 17, wobei das geschädigte Gewebe oder Organ ausgewählt ist aus der Liste umfassend: Haut, Blase, Harnröhre, Hornhaut, Schleimhaut, Bindehaut, Bauchwand, Bindehaut, Trommelfell, Rachen, Kehlkopf, Darm, Bauchfell, Band, Sehne, Knochen, Hirnhaut oder Vagina.

19. Arzneimittel, das das künstliche Gewebe nach Anspruch 14 umfasst.

## Revendications

1. Procédé *in vitro* pour la préparation d'un tissu artificiel comprenant :
a) l'addition d'une composition comprenant du fibrinogène à un échantillon de cellules isolées,
b) l'addition d'un agent antifibrinolytique au produit résultant de l'étape (a),
c) l'addition au moins d'un facteur de coagulation, d'une source de calcium, de thrombine, ou de toute combinaison de ce qui précède au produit résultant de l'étape (b),
d) l'addition d'une composition d'un polysaccharide au produit résultant de l'étape (c),
e) la culture de cellules isolées dans ou sur le produit résultant de l'étape (d), et
f) l'induction de la nanostructuration du produit résultant de l'étape (e), ladite induction de nanostructuration comprenant la déshydratation et/ou la compression mécanique du produit résultant de l'étape (e), le polysaccharide de l'étape (d) étant l'agarose.

2. Procédé selon la revendication 1, dans lequel les cellules de l'étape (a) sont des fibroblastes ou des kératocytes.

3. Procédé selon l'une quelconque des revendications 1 et 2, comprenant en outre une étape (étape b2) entre les étapes (b) et (c) dans laquelle une protéine est ajoutée.

4. Procédé selon la revendication 3, dans lequel la protéine ajoutée à l'étape (b2) est la fibronectine.

5. Procédé selon l'une quelconque des revendications 1 à 4, comprenant une étape (d2) entre les étapes (d) et (e) qui comprend l'addition d'une composition comprenant une protéine au produit résultant de l'étape (d).

6. Procédé selon la revendication 5, dans lequel la protéine ajoutée à l'étape (d2) est le collagène.

7. Procédé selon l'une quelconque des revendications 1 à 6, dans lequel les cellules de l'étape (e) comprennent des cellules souches de cordon ombilical.

8. Procédé selon l'une quelconque des revendications 1 à 7, dans lequel les cellules de l'étape (e) comprennent des cellules épithéliales.

9. Procédé selon la revendication 1, dans lequel la déshydratation du produit résultant de l'étape (e) comprend un procédé choisi dans la liste comprenant : un égouttage, une évaporation, une succion, une pression capillaire, une osmose ou une électro-osmose.

10. Procédé selon la revendication 9, dans lequel la déshydratation du produit résultant de l'étape (e) au moyen de pression capillaire comprend l'application d'une matière absorbante sur le produit résultant de l'étape (e).

11. Procédé selon l'une quelconque des revendications 1 à 10, dans lequel la compression mécanique de l'étape (f) comprend un procédé choisi dans la liste comprenant : une application d'une charge statique, une application d'un élément hydraulique, une application d'une came, une application d'un ou de plusieurs rouleaux, une application d'un ballon, une extrusion ou une centrifugation.

12. Procédé selon la revendication 11, dans lequel l'application d'une charge statique de l'étape (f) comprend le placement d'un poids sur le produit résultant de l'étape (e).

13. Procédé selon l'une quelconque des revendications 1 à 12, dans lequel se trouve, entre les étapes (e) et (f), une étape supplémentaire dans laquelle le produit résultant de l'étape (e) est exposé à l'air.

14. Tissu artificiel pouvant être obtenu par le procédé selon l'une quelconque des revendications 1 à 13.

15. Utilisation du tissu artificiel selon la revendication 14, pour l'évaluation d'un produit pharmacologique et/ou chimique.

16. Tissu artificiel selon la revendication 14 destiné à être utilisé en médecine.

17. Tissu artificiel selon la revendication 14, destiné à être utilisé pour augmenter, restaurer ou remplacer partiellement ou complètement l'activité fonctionnelle d'un tissu ou organe malade ou endommagé.

18. Tissu artificiel destiné à être utilisé selon la revendication 17, l'organe ou le tissu endommagé étant choisi dans la liste comprenant : la peau, la vessie, l'urètre, la cornée, une muqueuse, la conjonctive, la paroi abdominale, la conjonctive, le tympan, le pharynx, le larynx, l'intestin, le péritoine, un ligament, un tendon, un os, une méninge ou le vagin.

19. Composition pharmaceutique comprenant le tissu artificiel selon la revendication 14.
